# EUROPÄISCHE PATENTSCHRIFT

(11) **EP 1 029 892 B1**
(45) Veröffentlichungstag und Bekanntmachung des Hinweises auf die Patenterteilung: **05.06.2002**
(21) Anmeldenummer: 99102499.3
(22) Anmeldetag: 10.02.1999
(51) Int. Cl.: C08L 5/00, A61L 15/28, A61K 9/20

(54) **Beta-1,3-Glucan aus Euglena enthaltendes gefriergetrocknetes Erzeugnis, seine Herstellung und Verwendung**
Freeze-dried product containing beta-1,3-glucan from Euglena, its preparation and use
Produit lyophilisé contenant du beta-1,3-glucane issu d'Euglena, sa production et son utilisation

(43) Veröffentlichungstag der Anmeldung: 23.08.2000
(73) Patentinhaber: Dr. Suwelack Skin & Health Care AG, 48727 Billerbeck (DE)
(72) Erfinder: Bäumer, Dietrich Dr., 32139 Spenge (DE); Kahmann, Uwe Dr., 33813 Oerlinghausen (DE); Suwelack, Wolfgang, 48723 Billerbeck (DE); Tewes-Schwarzer, Petra, 48308 Senden (DE)
(74) Vertreter: Sieckmann, Ralf, Dr.

(56) Entgegenhaltungen:
- EP-A- 0 792 653
- WO-A-99/01166
- DE-A- 4 328 329
- DE-U- 29 723 220
- US-A- 4 774 093

## Beschreibung

Die vorliegende Erfindung betrifft ein Beta-1,3-Glucan aus Euglena enthaltendes gefriergetrocknetes Erzeugnis zur parenteralen oder peroralen Verabreichung, ein Verfahren zur Herstellung des vorgenannten Erzeugnisses sowie die Verwendung des Erzeugnisses als Nahrungsergänzungsmittel und Verabreichung von Kosmetika oder Pharmazeutika. Im folgenden wird Paramylon und Beta-1,3-Glucan aus Euglena synonym verwendet.

Aus der EP-B 417 254 = US-A 5 385 832 ist die Herstellung und Weiterverarbeitung eines aus *Euglena*-Zellen gewonnenen β-1,3-Glucans bekannt, bei dem nach dem Kultivieren die Zellen vom Kulturüberstand getrennt, die Zellen zerstört und mittels eines organischen Lösemittels extrahiert, Zellmasse abgetrennt und das erhaltene β-1,3-Glucan gewaschen wird. Dieses β-1,3-Glucan soll in der Medizin ebenso eingesetzt werden können wie in der Kosmetik oder als Lebensmittel. Das erfindungsgemäße Herstellverfahren unterscheidet sich hiervon durch die Vermeidung von giftigen organischen Extraktionsmitteln, wie Methanol und Chloroform, und liefert das β-1,3-Glucan Paramylon bereits innerhalb weniger Tage in Reinheiten weit oberhalb des durch eine lösemittelhaltige Extraktion erzeugten Glucans von nur oberhalb 95 bis 97 %, nämlich von meist mehr als 99 bis 99,5 Gew.-%. Eine Nachstellung des einzigen Ausführungsbeispiels, wie nachstehend diskutiert, zeigte weiter,aufgrund der fehlenden essentiellen Aminosäuren, ein für ein technisch realistisches Kultivierungsverfahren völlig unzureichendes Zellwachstum.

Die DE-A 43 28 329 betrifft eine gefriergetrocknete Biomatrix zur topischen Applikation, die 10 bis 80 Gew.-% natürliche und 20 bis 90 Gew.-% modifizierte Polysaccharide enthält.

Aus der EP-A-0 317 079 ist ein Mittel bekannt, bei dem die Inhaltsstoffe praktisch ohne Verbund, jedoch in gepreßter Form in Kapseln eingebracht oder zu Tabletten verpreßt werden. Nach dem Einnehmen löst sich die Kapsel bzw. die gepreßte Tablette im Magen auf, die Inhaltsstoffe bilden eine gequollene Masse, die im Magen befindliche Flüssigkeit bindet und wird über den Verdauungstrakt abgebaut bzw. wieder ausgeschieden. Da das Mittel keinerlei Brennwert hat, dem Körper jedoch ein Sättigungsgefühl aufgrund der im Magen befindlichen Quellmasse vorgetäuscht wird, wird die Nahrungsaufnahme ohne große Überwindung zum Zweck der Gewichtsreduktion eingestellt bzw. vermindert werden. Da die Quellmasse im wesentlichen ungebunden ist, wird sie relativ schnell aus dem Magen in den weiteren Verdauungstrakt abgeleitet, weshalb das dadurch hervorgerufenen Sättigungsgefühl nur vergleichsweise kurze Zeit anhält.

Die DE 29 723 220 betrifft ein Mittel zur peroralen Verabreichung, enthaltend wenigstens einen komprimierten, nicht toxischen Träger, der wenigstens zum Teil über den Verdauungstrakt abbaubar, abgebaut, abgeführt und/oder abführbar ist, wobei der Träger nach der Expansion im Magen eine schwammartige und wenigstens teilweise eine Collagenstruktur aufweist.

Die EP-A 0 202 159 beschreibt eine Vorrichtung aus wenigstens einem im Magen lösbaren Polymer und wenigstens einem nicht löslichen Polymer, wobei das nicht lösbare Polymer u.a. aus nicht wasserlöslichen Cellulosen ausgewählt ist.

Die EP-A-0 471 217 (und teilweise entsprechend die prioritätsbegründende DE-A 40 25 912) betrifft ein Mittel zur oralen Einnahme mit einem im Magen lösbaren und den Inhalt freigebenden Behältnis, das mit einem beim Freisetzen volumenvergrößernden, nicht toxischen und brennstoffarmen Stoff gefüllt ist, der innerhalb des Verdauungstraktes abbaubar oder über diesen abführbar ist, wobei der Stoff ein Schwamm ist, der in komprimierter Form im Behältnis angeordnet ist und durch das Behältnis in dieser komprimierten Form gehalten wird. Dieser besagte brennstoffarme Stoff ist vorzugsweise ein Cellulose-Schwamm oder ein Polyurethan-Schaumstoff. Dieses vorgenannte Verfahren ist allerdings in der Praxis wenig ausführbar, da der Einsatz eines Polyurethan-Schaumstoffs als komprimierter, expandierbarer Stoff sowohl in der Bundesrepublik Deutschland als auch in weiteren ausländischen Staaten nach der Zusatzstoff-Rahmen-Richtlinie 89/107/EWG bzw. der Richtlinie über sonstige Zusatzstoffe in der EG nicht zugelassen ist. Darüber hinaus scheint auch die spezielle erstmals in der Nachanmeldung angesprochene Schwamm- oder Aveolar-Cellulose wenigstens nach den lebensmittelrechtlichen Bestimmungen nicht zugelassen, da dort ausschließlich mikrokristalline Cellulose und Pulvercellulose, Methylcellulose, Hydroxypropylcellulose, Hydroxypropylmethylcellulose, Methylethylcellulose und Natriumcarboxymethylcellulose zugelassen sind. Auch ist das in der vorgenannten Patentanmeldung beschriebene Mittel insofern technisch aufwendig gestaltet, als es als zwingenden Bestandteil stets ein im Magen lösbares und den Inhalt freigebendes Behältnis, also konkret eine Hartgelatinekapsel voraussetzt.

Der vorliegenden Erfindung liegt die Aufgabe zugrunde, ein Beta-1,3-Glucan enthaltendes Erzeugnis zur parenteralen oder peroralen Verabreichung bereitzustellen, das gegenüber den vorgenannten einfachen Quellmassen oder Quellmassen in Schwammform technisch einfacher und in hoher Reinheit hergestellt wird, und insbesondere lebensmittelrechtlich zugelassen ist.

Diese Aufgabe wird durch den Einsatz eines 0,1 bis 100 Gew.-% Beta-1,3-Glucan aus Euglena enthaltenden gefriergetrockneten Erzeugnisses gelöst.

Die vorliegende Erfindung betrifft somit ein Erzeugnis, enthaltend 0,1 bis 100 Gew.-% Beta-1,3-Glucan aus Euglena, wobei das Beta-1,3-Glucan aus Euglena erhalten worden ist durch Kultivierung von Euglena-Zellen in einem Kulturmedium, Abtrennung der Euglena-Zellen aus dem Kulturmedium, Isolierung des Beta-1,3-Glucan aus Euglena aus den Euglena-Zellen, Reinigung des Beta-1,3-Glucan aus Euglena, gegebenenfalls unter Zusatz von modifizierter Polysaccharide sowie gegebenenfalls biologisch aktiver Wirkstoffe, Abkühlung und anschließende Gefriertrocknung und wobei die Isolierung des Beta-1,3-Glucan aus Euglena aus den Euglena-Zellen durch Zugabe wenigstens eines Tensids erfolgt.

Das vorgenannte gefriergetrocknete Beta-1,3-Glucan aus Euglena wird in an sich bekannter Weise erhalten. Wir verweisen z.B. auf E. Ziegler, die natürlichen und künstlichen Aromen, Heidelberg, 1982, Kapitel 4.3, Gefriertrocknen und die dort angegebene Literatur sowie auf die DE-A 43 28 329.

Die Erfindung betrifft weiter ein Verfahren zur Herstellung des gefriergetrockneten Beta-1,3-Glucans aus Euglena / Trägers der vorbeschriebenen Art durch Kultivierung von Euglena-Zellen in einem Kulturmedium, Abtrennung der Euglena-Zellen aus dem Kulturmedium, Isolierung des Beta-1,3-Glucan aus Euglena aus den Euglena-Zellen, Reinigung des Beta-1,3-Glucan aus Euglena, wobei es weiterhin die Umsetzung des Beta-1,3-Glucan aus Euglena, gegebenenfalls modifizierter Polysaccharide sowie gegebenenfalls biologisch aktiver Wirkstoffe, Abkühlung und anschließende Gefriertrocknung umfaßt und die Isolierung des Beta-1,3-Glucan aus Euglena aus den Euglena-Zellen durch Zugabe wenigstens eines Tensids erfolgt.

Unter *Euglena*-Zellen im Sinne der vorliegenden Erfindung können jegliche Spezies eingesetzt werden, beispielsweise *Euglena gracilis, Euglena intermedia, Euglena piride* und andere Euglenoide, z. B. *Astaia longa*. Bevorzugt ist der Einsatz von *Euglena-gracilis*.

Hierbei ist es bevorzugt, daß die Isolierung des Beta-1,3-Glucan aus Euglena aus den *Euglena*-Zellen ohne Zusatz organischer Lösungsmittel erfolgt.

Obgleich durch das vorgenannte Verfahren sämtliche Algen der Klasse *Euglena* eingesetzt werden können, wie sie beispielsweise auch in der vorgenannten EP-B 417 254 im einzelnen erläutert sind, ist es bevorzugt, als Alge insbesondere *Euglena*-*gracilis*-Zellen einzusetzen.

Weiterhin ist es bevorzugt, daß der im Rahmen des erfindungsgemäßen Erzeugnisses eingesetzte Träger durch eine Kultivierung der *Euglena*-Zellen als Fed-Batch-Kultivierung durchgeführt wird.

Gemäß einer weiteren bevorzugten Ausführungsform wird das für den späteren Einsatz als Träger verwendete Beta-1,3-Glucan aus Euglena aus den *Euglena*-Zellen mittels eines weitgehend biologisch abbaubaren Tensides isoliert, welches ausgewählt ist aus nicht-ionischen oder anionischen Tensiden.

Als anionische Tenside kommen Sulfonate, wie beispielsweise Alkylbenzolsulfonat, Alkansulfonate, α-Olefinsulfonate, Sulfofettsäureester, Sulfobernsteinsäureester, Sulfosuccinamate, Acyloxyalkansulfonate, Acylaminoalkansulfonate, Sulfate, Carboxylate, Alkylethersulfate, Alkylarylethersulfate und Alkylethercarboxylate in Betracht.

Als nicht-ionische Tenside kommen beispielsweise Polyglykolether, wie Alkylpolyglykolether, Alkylarylpolyglykolether, Acylamidpolyglykolether, Alkylaminpolyglykolether, Ethylenoxid-Propylenoxid-Addukte wie Alkyl-Ethylenoxid-Propylenoxid-Adukte, Polypropylenoxid-Polyethylenoxid-Addukte, trifunktionelle Ethylenoxid/Propylenoxid-Addukte, tetrafunktionelle Ethylenoxid/Propylenoxid-Addukte, Polyolester wie beispielsweise Zuckerester, die auch unter der Bezeichnung Alkylpolyglykosid bekannt sind, Polyolpolyglykolether, Fettsäurealkanolamide, Fettsäuremonoethanolamide, Fettsäurediethanolamide und Aminoxide.

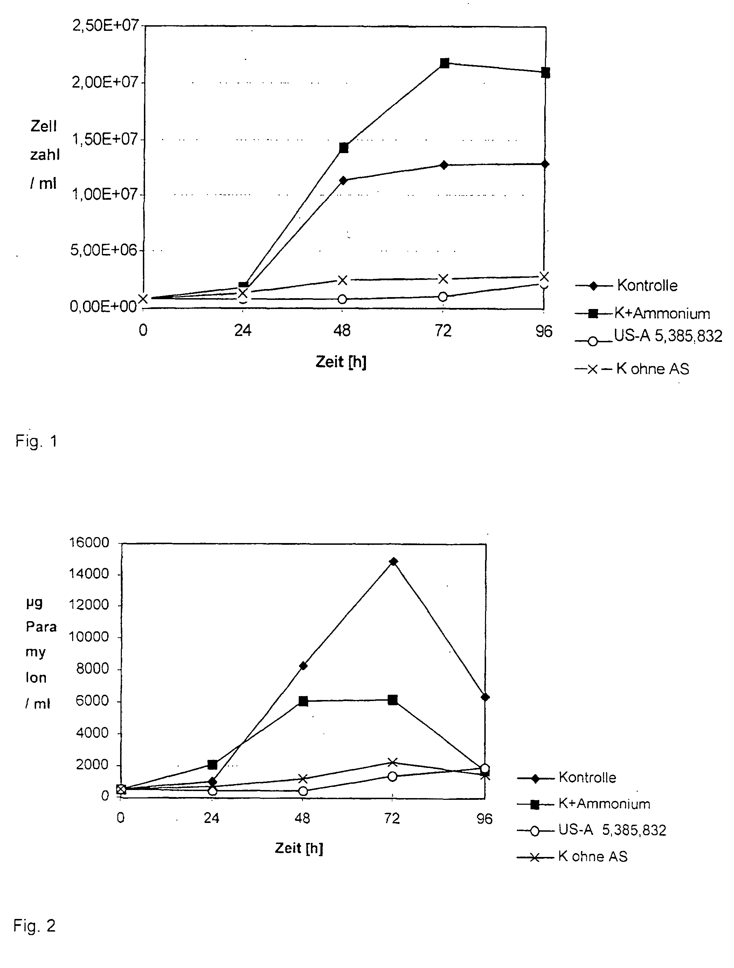

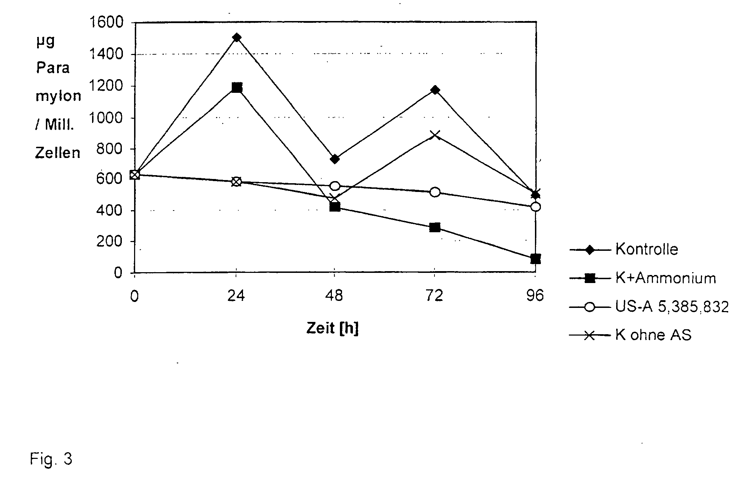

In der US-A 5,385,832 wird nun im Ausführungsbeispiel ein Kulturmedium beschrieben, das als Stickstoffquelle Ammoniumsulfat verwendet und zwar in Konzentrationen von mehr als 1,9 g/Liter. Dies entspricht einer Ammoniumkonzentration von mehr als 600 mg/ Liter, die *Euglena* bereits im Wachstum und auch bei der Paramylonsynthese empfindlich stört. Im Medium fehlen die Aminosäuren, von denen einige, wie erfindungsgemäß gezeigt werden konnte, essentiell sind. Es sind dies die Aminosäuren Aspartat, Glutamat und Glycin. Fehlt eine dieser Aminosäuren, so ist sowohl das Wachstum von *Euglena,* als auch die Paramylonsynthese gehemmt. Weiterhin ist es ein bekanntes Phänomen, daß *Euglena* unter Ammoniumstreß das gebildete Paramylon wieder abbaut. Dies wurde bereits von S. Sumiida et. al. in *Plant and Cell Physiology* 28 (8) 1987, 1587, insbesondere Fig. 1 und Fig. 3 belegt. In dem erfindungsgemäß zur Herstellung des Paramylons verwendeten Kulturmediums befindet sich daher kein Ammoniumsulfat. Die Ammoniumkonzentration liegt bei dem erfindungsgemäß eingesetzten Teilverfahrensschritt zu Beginn der Fermentation meist im Bereich von 100 - 250 mg/l und stammt lediglich aus dem Zerfall von Aminosäuren und dem zugesetzten Ammoniumeisen-(II)-sulfat.

Die Zusammensetzung des zur Herstellung des erfindungsgemäß verwendeten Mediums ist im folgenden aufgeführt:

| | |
|---|---|
| Vitamin B₁ (Thyamin) | 0,3 - 1,0 mg/l |
| Vitamin B₁₂ | 0,01 - 0,1 mg/l |
| KH₂PO₄ | 0,2 - 0,6 g/l |
| MgSO₄ x 7H₂O | 0,05 - 0,3 g/l |
| Fe(SO₄)₂(NH₄)₂ x 6 H₂O | 10 - 30 mg/l |
| ZnSO₄ x 7 H₂O | 5 - 20 mg/l |
| MnSO₄ x H₂O | 2 - 10 mg/l |
| (NH₄)₆Mo₇O₂₄ x 4 H₂O | 0,5 - 5 mg/l |
| CoSO₄ x 7H₂O | 0,1 - 0,5 mg/l |
| CuSO₄ x 5H₂O | 0,3 - 1,0 mg/l |
| H₃BO₃ | 0,1 - 0,5 mg/l |
| NaNO₃ x 4H₂O | 0,1 - 1 mg/l |
| MgCO₃ | 0,1 - 0,8 g/l |
| Glucose | 5 -15 g/l |
| Harnstoff | 0,1 - 0,6 g/l |
| L-Glutaminsäure | 4-15 g/l |
| D,L-Asparaginsäure | 1 - 5 g/l |
| Glycin | 2 - 6 g/l |
| D,L-Äpfelsäure | 3 - 6 g/l |
| Natriumsuccinat x 6H₂O | 0,05 - 0,5 g/l |
| CaCO₃ | 0,05 - 0,5 g/l |

Dieses Medium wird bei den im folgenden beschriebenen Wachstumsversuchen als Kulturmedium (K) bezeichnet.

Die vorliegende Erfindung wird nachstehend durch Figuren näher gekennzeichnet.

Es zeigen:
Fig. 1 das Wachstumsverhalten der heterotrophen *Euglena gracilis* in den verschiedenen Kulturmedien.
Fig. 2 den Paramylongehalt in *Euglena gracilis* im Verlauf der Fermentation über 96 h in verschiedenen Kulturmedien.
Fig. 3 den Paramylongehalt in *Euglena* während der Fermentation in den 4 Kulturmedien, bezogen auf die Zellzahl von 1x 10⁶.

Die Fig. 1 bis 3 zeigen im Vergleich das Wachstum von *Euglena gracilis* und den Paramylongehalt bei der Kultivierung der Zellen in verschiedenen Medien:
1. das erfindungsgemäß zur Paramylonherstellung eingesetzte Kulturmedium (**K**),
2. das im Ausführungsbeispiel des US-Patent US-A 5,385,832 = EP-B 417 254 beschriebene Kulturmedium (Vergleichsbeispiel: US-Patentmedium)
3. das erfindungsgemäß zur Paramylonherstellung eingesetzte Kulturmedium, dem aber noch Ammoniumsulfat zugesetzt wurde, zur Erhöhung der Ammoniumkonzentration auf 600 -700 mg/l (K+Ammonium),
4. das erfindungsgemäß zur Paramylonherstellung eingesetzte Kulturmedium, in dem die Aminosäuren fehlen (K ohne AS).

Fig. 1 zeigt deutlich, daß die Zellen im US-Patentmedium während der Fermentation über 96 h nicht über eine Zellzahl von 2,5 x 10⁶ /ml hinauskommen. Gleiches gilt für das Medium, dem die Aminosäuren fehlen. Die Zellen im US-Patentmedium sind weiterhin stark vakuolisiert, ein Zeichen für Mangel und den Versuch, Ammonium zu "entsorgen". Die Zellen enthalten fast keine Paramylongranula. Die Kontrollkultur erreicht Zellzahlen von etwa 14 x 10⁶/ml, wobei die Kultur im dargestellten Versuch nicht mit Nährstoffen supplementiert wurde. Das zusätzlich zum Kontrollmedium zugegebene Ammonium führt jedoch zu einer offensichtlichen Steigerung des Wachstums der Zellen. Dies bedeutet aber, daß die Energie überwiegend in die Zellteilung investiert wird und nicht in die Paramylonsynthese, wie die folgende Abbildung 2 deutlich macht.

Gemäß Fig. 2 bauen die Zellen durch die hohe Ammoniumkonzentration das Paramylon nach 72 h ab. Aber selbst nach 72 h sind insgesamt nur etwa 6 mg Paramylon/ml Kultur in den Ammonium supplementierten Zellen nachweisbar, während bei der Kontrollkultur etwa 15 mg Paramylon/ml Kultur nachweisbar sind. Auch bei der erfindungsgemäßen Kontrollkultur setzt nach 72 h der Abbau des Paramylons ein, jedoch aufgrund der Tatsache, daß die Kohlenstoffquelle erschöpft ist. Würde jedoch nach 72 h mit Glucose und Aminosäuren supplementiert, so würden Zellzahl und Paramylongehalt weiter ansteigen. Dies ist bei der Ammonium-Kultur jedoch nicht der Fall, bei ihr liegt nach 72 h noch reichlich Glucose im Medium vor (7-8 g/l). Die Kontrollkultur ohne Aminosäuren sowie die US-Patent-Kultur synthetisieren kaum Paramylon, wie Fig. 1 deutlich zu entnehmen ist. Zu erwähnen wäre noch, daß die Zellen, die im US-Patentmedium kultiviert wurden, trotz Nährstoffzufuhr nach etwa 10 bis 12 Tagen absterben.

Fig. 3 zeigt deutlich, daß der Paramylongehalt in der US-Patent-Kultur sowie in dem ammoniumsupplementierten Kontrollmedium im Verlauf der Fermentation im Bezug auf die Zellzahl abnimmt. Die Kontrollkultur, sowie die Kontrolle ohne Aminosäuren zeigen die durch die Zellteilung bedingten Schwankungen im Paramylongehalt pro Zelle. Bei jeder Zellteilung werden die Paramylongranula natürlich auf die Tochterzellen verteilt, dies führt zu einer vorübergehenden Abnahme des Paramylongehaltes pro Zelle. Die Kultur ohne Aminosäuren synthetisiert jedoch deutlich geringere Paramylonmengen als die Kontrollkultur.

Die Auswertung der Figuren ergibt, daß in den Kulturen mit hoher Ammoniumkonzentration erfindungsgemäß unerwünscht das Paramylon abgebaut wird. Durch das Fehlen lebensnotwendiger Aminosäuren ist in den US-Patentkulturen gemäß Stand der Technik kaum ein Zellwachstum zu verzeichnen und die Zellen sterben nach einiger Zeit ab. Bei der Kontrollkultur stehen jedoch innerhalb der ersten 72 h der Kultivierung Zellwachstum und Paramylongehalt in einem optimalen Verhältnis zueinander. Die Zellen sind prall mit Paramylon gefüllt. Dies ermöglicht eine leichte Isolierung, aber es sorgt vor allem für eine höhere Reinheit des isolierten Paramylons, da der Anteil an Protein und Lipid, bezogen auf die Paramylonmenge, geringer ist.

Im Gegensatz zu anderen Trocknungsverfahren, wie beispielsweise der Sprühtrocknung, Lufttrocknung liefert das Verfahren der Gefriertrocknung schwammähnliche, schnell rehydratisierbare Materialien mit einem Convenience-Charakter (Instanteffekt), das heißt, es bildet sich bei Feuchtigkeitszufuhr spontan eine Art Gel aus.

Nach einer bevorzugten Ausführungsform der vorliegenden Erfindung enthält das Erzeugnis 3 bis 100 Gew.-% Beta-1,3-Glucan aus Euglena.

Nach einer weiteren bevorzugten Ausführungsform enthält das Erzeugnis 0,1 bis 99 Gew.-%, vorzugsweise 5 bis 95 Gew.-% Beta-1,3-Glucan aus Euglena und 1 bis 99,9 Gew.%, vorzugsweise 5 bis 95 Gew.-% eines weiteren Trägers, ausgewählt aus natürlichen Polysacchariden und/oder modifizierten Polysacchariden und/oder Collagen.

Nach einer weiteren bevorzugten Ausführungsform enthält das Erzeugnis 1 bis 99 Gew.-%, vorzugsweise 5 bis 95 Gew.-%, Beta-1,3-Glucan aus Euglena und 1 bis 99 Gew.%, vorzugsweise 5 bis 95 Gew.-%, des weiteren Trägers Collagen.

Die vorgenannten natürliche Polysaccharide werden vorzugsweise aus der Gruppe bestehend aus Pektinen, Alginaten, Carrageen, Agar-Agar und Johannisbrotkernmehl ausgewählt.

Als modifizierte Polysaccharide, die als Bestandteil des vorgenannten Trägers mit eingesetzt werden können, kommen beispielsweise Cellulosederivate, beispielsweise Celluloseether in Betracht. Bevorzugt sind filmbildende Bindemittel, wie beispielsweise Carboxymethylcellulose oder deren Derivate. Carboxymethylcellulose kann mit anderen Celluloseethem, Polyestern oder Polyvinylalkohol in vorteilhafter Weise kombiniert werden.

Nach einer bevorzugten Ausführungsform kann das Erzeugnis weiterhin Spinnfasern, Calziumionen, kosmetisch und pharmazeutisch aktive Wirkstoffe und micellenbildende Stoffe enthalten.

Die erfindungsgemäß als Trägerbestandteil eingesetzten Polysaccharide können in vorteilhafterweise mit Proteinen pflanzlichen Ursprungs kombiniert werden. Beispiele hierfür sind Sojaproteine oder Proteine von Zerealien. Weiterhin können jedoch auch zusätzlich Polysaccharide aus der Gruppe der Glykosaminoglykane wie Hyaluronsäure, deren Derivate und Chondroitinsulfat verwendet werden.

Neben den vorgenannten natürlichen oder modifizierten Polysacchariden kann das erfindungsgemäße Erzeugnis Spinnfasern zur Verbesserung der Trockenstabilität und biologische Wirkstoffe, insbesondere kosmetische und pharmazeutische Wirkstoffe enthalten.

Nach einer weiteren bevorzugten Ausführungsform der vorliegenden Erfindung enthält es micellenbildenden Stoffe, beispielsweise Isoparaffine, die typischerweise in einem Gesamtanteil im Mittel von 0,1 bis 1,5 Gew.-% und insbesondere von 1,15 bis 1,8 Gew.-% vorliegen.

Die als Bestandteil des Trägers im erfindungsgemäßen Erzeugnis eingesetzten Polysaccharide sind vorzugsweise pflanzlichen Ursprungs und zeichnen sich funktionell durch schutzkolloide Eigenschaften aus. Als modifizierte Polysaccharide kommen alle filmbildenden Bindemittel in Betracht, die einerseits eine besondere Affinität zu den natürlichen Polysacchariden und andererseits zu den gegebenenfalls eingesetzten Spinnfasern haben. Die Verwendung von Carboxycellulose bietet den Vorteil, daß es sich um ein reversibel wasserlösliches Produkt handelt, welches nicht-toxisch und international als kosmetischer Grund- und Hilfsstoff (Binde- und Verdickungsmittel, Schutzkolloid) zugelassen ist. Carboxymethylcellulose gestattet zudem die Kombination mit anderen Celluloseethern in vorteilhafter Weise, so daß unterschiedliche Qualitäten gefriergetrockneter Biomatrices hergestellt werden können.

Als Spinnfasern kommen Celluloseester-, Polyester-, Polyamid-, Polyvinylalkohol-, Woll-, Baumwoll-, Seiden- und Viskosefasern in Betracht, wobei Viskosefasern besonders bevorzugt sind. Vorteilhafterweise besitzen die Spinnfasern eine Länge von 3 bis 30 mm und einen Titer von 1 bis 6 dtex (1 dtex = 7.85 x 10⁻³ pd²; p = Dichte in g/cm³, d = Durchmesser in µm).

In einer bevorzugten Ausführungsform der vorliegenden Erfindung sind in dem beta-1,3-Glucan aus Euglena enthaltenden gefriergetrockneten Erzeugnis 3 bis 30 Gew.-% und insbesondere 3 bis 15 Gew.-% Spinnfasern enthalten.

Die bevorzugte Verwendung von Viskosefasern beruht auf der Tatsache, daß es sich um die hydrophilsten der üblicherweise verfügbaren Fasern handelt, die deshalb für die Herstellung von löslichen Produkten am besten geeignet sind. Viskosefasern sind, ebenso wie die weiteren bevorzugten Bestandteile des Trägermaterials, im erfindungsgemäßen Erzeugnis nicht-toxische modifizierte Polysaccharide. Aus diesem Grund besitzen sie sehr hohe Affinität zu den übrigen Bestandteilen, was zur Folge hat, daß bereits eine kleine Menge dieser Spinnfasern zur Stabilisierung des Trägers beiträgt. Die bevorzugt verwendete Viskose ist sowohl für kosmetische als auch für medizinische Zwecke bzw. Anwendungen zugelassen.

Als kosmetisch aktive Substanzen kommen eine Vielzahl von Produkten in Betracht, beispielsweise Vitamine, Proteine, wasserlösliche Pflanzenextrakte und Liposomen.

Ein schneller perkutaner Transport von Hautwirkstoffen, die proportional zum Durchfeuchtungsgrad des Stratum corneum gefördert wird, kann durch die Verwendung der gefriergetrockneten paramylonenthaltenden Mittel, deren Hauptbestandteile selbst hautbefeuchtende Wirkung besitzen, gefördert werden. Dabei diffundieren kleine Moleküle, die dem Träger oder der wäßrigen Lösung zugesetzt werden, aus dem konzentrierteren Bereich des mit Wasser übersättigten Mittels in den schwächer konzentrierten Bereich der Epidermis. Zu den relativ leicht penetrationsfähigen Molekülen zählen Substanzen wie kurzkettige Peptide, ATP, Harnstoff und Elektrolyte.

Die vorbeschriebenen idealen perkutanen Transportbedingungen gelten andererseits auch für diejenigen niedermolekularen Substanzen, deren Penetration durch die Hornhautbarriere unerwünscht und durchaus bedenklich ist, weil sie Hautirritationen auslösen können. Zu den unerwünschten Substanzen mit nachgewiesenem Irritationspotential zählen beispielsweise bekannte Kosmetikkonservierungs- und Parfumierungsmittel sowie oberflächenaktive Substanzen, wie sie üblicherweise in marktgängigen Emulsionsprodukten Verwendung finden.

Die erfindungsgemäßen gefriergetrockneten paramylonenthaltenden Erzeugnisse zeichnen sich dadurch aus, daß weitgehend und vorzugsweise vollständig auf die Verwendung der obengenannten Stoffe verzichtet werden kann.

Im Gegensatz zu herkömmlichen Xerogelen, bei denen sich nach Wasserentzug die räumliche Anordnung des Netzes (Matrix) so verändert, daß die Abstände zwischen den Strukturelementen nur die Größenordnung von Atomabständen besitzen, bilden die erfindungsgemäßen gefriergetrockneten paramylonenthaltenden Erzeugnisse in Gelform Hohlräume aus, in die die Lösungsmittelflüssigkeit schnell und ungehindert eindringen und den spontanen Zerfall des Netzwerkes in situ herbeiführen kann, die sogenannte Instantgelbildung. Das Verfahren der Gefriertrocknung führt, ähnlich wie bei der Entfernung von Wasser durch Sublimation aus Nahrungsmittelrohstoffen, wie zum Beispiel Tee oder Kaffee, zu Kapillarstrukturen, die sich sehr schnell rehydratisieren lassen und den gewünschten Instanteffekt ermöglichen.

Bei der Verwendung von Substanzen mit vielen polaren Gruppen, beispielsweise Cellulose und Proteinen, neigt das wäßrige Lösungsmittel zu starken Brückenbindungen zwischen diesen Gruppen, wodurch das Lösungs- und Diffusionsverhalten beeinflußt wird. Bei Zufügen von genügend Wasser über die Ausbildung von Brückenbindungen hinaus wird die Polymermatrix zum Quellen gebracht und es wird damit sichergestellt, daß eine große Mobilität der Wasseratome und der darin enthaltenden Wirkstoffe erzielt wird. Dieser bei den erfindungsgemäßen gefriergetrockneten Erzeugnissen beobachtete Effekt ist im Hinblick auf die kosmetische Anwendung und das Desorptions- und Penetrationsverhalten der kosmetischen Wirkstoffe von größter Bedeutung. Die erfindungsgemäßen gefriergetrockneten Erzeugnisse ermöglichen ferner die Ausbildung von feuchtigkeitsstabilen Matrixformen, wenn das als Lösungsmittel zugefügte Wasser Calcium-Ionen in einer Menge enthält, die ausreicht, um Natriumionen beispielsweise aus Alginsäuresalzen ganz oder teilweise auszutauschen. Das spontan gebildete Calcium-Alginat-Gerüst stabilisiert die erfindungsgemäßen Erzeugnisse in einem solchen Maße, daß jeweils nur ein vorbestimmter Teil der Polymere in den Gelzustand übergehen kann.

Durch die Zugabe von hydrophilem Fasermaterial, beispielsweise Viskosefasern, wird eine weitere Stabilisierung der Matrix ermöglicht.

In jedem Fall kann durch die wahlweise Verwendung von Spinnfasern oder durch Stabilisierung mit Calcium-Ionen die Konsistenz der erfindungsgemäßen gefriergetrockneten Mittel gezielt eingestellt werden und ermöglicht so eine Anpassung des Produkts an das jeweilige gewünschte Anwendungsgebiet, wobei die erforderliche einfache und zweckmäßige Handhabung gewährleistet ist.

Nach einer weiteren bevorzugten Ausführungsform werden dem erfindungsgemäßen gefriergetrockneten paramylonenthaltenden Erzeugnis Stoffe zugemischt, die zur Micellenbildung befähigt sind. Diese Substanzen liegen in dem erfindungsgemäßen gefriergetrockneten Erzeugnis mit einem Anteil von 4 bis 30 Gew.-%, vorzugsweise 5 bis 20 Gew.-%, vor. Bei diesem instantisierfähigen Mitteln bzw. Biomatrices pflanzlicher Herkunft werden als micellenbildende Stoffe beispielsweise Isoparaffine verwendet, die durch Micellbildung ein zusammenhängendes Gerüst aufbauen können, das die Herstellung von stabilen Gelen erlaubt.

Weiter bevorzugt ist es, daß die erfindungsgemäßen Mittel keine Parfum-, Farb- und Konservierungsstoffe enthalten.

Die erfindungsgemäßen gefriergetrockneten Substanzen können weiterhin kosmetische und/oder pflanzliche und/oder pharmazeutische Wirkstoffe, vorzugsweise mit einem Anteil von 0,1 bis 50 Gew.-% und insbesondere 3 bis 30 Gew.-% leicht eingearbeitet enthalten. Es ist von Vorteil, die Wirkstoffe in verkapselter Form einzugeben, wie beispielsweise in liposomalen und liposomenähnlichen Vesikeln.

### Herstellung des Erzeugnisses

Die erfindungsgemäßen gefriergetrockneten Erzeugnisse werden hergestellt, indem man zunächst neben dem Beta-1,3-Glucan aus Euglena die weiteren Träger, ausgewählt aus natürlichen Polysacchariden und/oder modifizierten Polysacchariden und/oder Collagen sowie gegebenenfalls die gewünschten kosmetisch oder pharmazeutisch aktiven Substanzen, einem wäßrigen Medium gleichmäßig miteinander vermischt und die Mischung anschließend kühlt. Während des Kühlens bildet sich das Gel. Anschließend werden diesem Gel gegebenenfalls die Spinnfasern schonend eingeführt und gleichmäßig verteilt. Nach Einrühren und erneutem Kühlen, beispielsweise auf etwa 1 °C, wird die Masse in Formen gegossen. In diesen Formen bildet sich die ursprüngliche Gelstruktur zurück und durch die anschließende Gefriertrocknung entsteht ein Material, das strukturell einem reinen Collagenschwamm ähnlich ist. Das Einfrieren ist als erste Phase der Ausbildung der späteren Matrix ein wesentlicher Verfahrensschritt, wobei beschleunigtes Einfrieren bei niedrigen Temperaturen erfindungsgemäß bevorzugt ist. Durch die anschließende Gefriertrocknung des tiefgefrorenen Gels im Hochvakuum wird das Lösemittel ausgefroren und verdampft (Sublimation). Ein wesentliches Kennzeichen der Gefriergetrocknung ist die Porenbildung ohne Volumenveränderung. Hierauf beruht der Effekt der schnellen Rehydratisierung, bekannt als Instanteffekt.

In einer bevorzugten Ausführungsform der vorliegenden Erfindung stellt man zunächst eine Mischung aus dem Beta-1,3-glucan aus Euglena und den weiteren Trägerbestandteilen her, die man in Wasser einrührt. Nach Kühlung der Mischung auf 10 °C kann in diese Vormischung ein Gemisch aus Spinnfasern, kosmetischen und/oder pharmazeutischen Wirkstoffen und/oder micellenbildenden Stoffen dispergiert werden. Die erhaltene Mischung wird bei - 10 °C bis - 40 °C, vorzugsweise bei etwa - 20 °C, über einen Zeitraum von 0,5 bis 4 Stunden, vorzugsweise 1 bis 3 Stunden, in Form von Platten eingefroren. Platten mit Schichtdicken von 0,5 bis 3,5 cm, vorzugsweise 1,5 bis 2,0 cm, sind dabei bevorzugt. Die Porengröße wird beim Einfrieren im wesentlichen über die Einfriergeschwindigkeit und die Temperaturcharakteristik gesteuert. Gegebenenfalls können die Platten bei - 10 °C bis - 25 °C zwischengelagert werden, bevorsie bei einer Heiztemperatur im Bereich zwischen 80 °C und 150 °C und einem Vakuum von etwa 0,5 bis 3,0 mbar gefriergetrocknet werden. Vorzugsweise führt man den Gefriertrocknungsprozeß über einen Zeitraum von etwa 15 bis 35 Stunden durch. Danach können die Platten gespalten und zugerichtet werden.

Nach dem Gefriertrocknungsprozeß liegt der Wassergehalt des nach dem oben dargestellten Verfahren erhaltenen gefriergetrockneten Trägermaterialien enthaltenen Erzeugnisses vorzugsweise im Bereich von 5 bis 15 %, wobei 10 % besonders bevorzugt ist. Die Trockenstoffkonzentration des zur Herstellung des erfindungsgemäßen gefriergetrockneten Erzeugnisses eingesetzten Ausgangsmaterials, das heißt der Mischung der Bestandteile im demineralisierten Wasser, beträgt etwa 1 bis 5 %.

### Peroral verabreichbare Erzeugnisse

Die vorliegende Erfindung betrifft somit weiter ein Erzeugnis zur peroralen Verabreichung, welches wenigstens einen paramylonenthaltenden gefriergetrockneten Träger komprimiert enthält, wobei der Träger nach der Expansion im Magen eine schwammartige Struktur aufweist

Unter den erfindungsgemäß als weiterem Trägerbestandteil eingesetzten Collagenstrukturen versteht man im wesentlichen die sogenannten Skleroproteine, die auch unter der Bezeichnung Faserproteine, Gerüstproteine oder Strukturproteine bekannt sind und eine Gruppe von wasserunlöslichen, faserförmig aufgebauten, tierischen Proteinen mit reiner Gerüst- und Stützfunktion darstellen. Das Collagen wird aus Stütz- und Bindegeweben, Haut, Knochen und Knorpeln gewonnen.

In einer weiteren bevorzugten Ausführungsform enthält der weitere Trägerbestandteil Collagen im erfindungsgemäßen Erzeugnis die Aminosäuren Glycin und Hydroxyprolin, vorzugsweise mit der Tripeptidsequenz GlyXy, wobei X für eine beliebige Aminosäure und anstelle von y häufig Hydroxyprolin steht.

Nach einer weiteren bevorzugten Ausführungsform stammt der weitere Bestandteil im Träger, Collagen, aus dem Stamm *Porifera,* insbesondere der Klasse *Demospon*giae. Hierbei handelt es sich um die zoologische Bezeichnung der umgangssprachlich als Schwamm bezeichneten Meerestiergruppe. Diese Meeresbewohner weisen eine ohne Symmetrie, jedoch polar organisierte klumpen-, krusten-, trichterbis schüssel-, aber auch pilz- und geweihförmige Gestalt auf, die durch ein Skelett aus Collagen-(Spongin) Fasern erzeugt wird, in das Skleren aus Calcit oder Kieselsäure eingelagert sind. Die Schwämme weisen meist drei Schichten auf, von denen die größte mittlere Schicht, das Mesohyl aus einer gallertartigen Grundsubstanz mit Collagenfasern besteht. Wir verweisen beispielsweise auf das Lexikon der Biologie, Band 7, Freiburg 1986, Stichwort Schwämme, sowie ebenda Band 8, Stichworte Spongia, Spongin.

Der Stamm *Porifera* gliedert sich in die Klassen *Calcarea*, d.h. Schwämmen mit Calciteinlagerungen, *Hexactinelliida*, also solche mit speziellen Kieselsäureeinlagerungen sowie *Demospongiae*, worunter solche mit einem Faser oder Kieselsäuregerüst fallen. Zur Gruppe der insbesondere geeigneten Klasse *Demospongiae*. Hierunter fallen insbesondere die Hornkieselschwämme (*Comacu-spongia*), die Süßwasserschwämme und der Badeschwamm (*Spongia officialis*) mit den Unterarten Levantinerschwamm (*Spongia officialis mollissima*), Zimmokaschwamm (*Spongia officialis cimmoca*), Elefantenohr (*Spongis offcialis lamella*) sowie der Großlöcherige Pferdeschwamm (*Hippospongia Communis*).

Die aus dem Wasser gewonnenen Schwämme werden in an sich bekannter Weise, beispielsweise durch sauren Aufschluß von den mineralischen Bestandteilen befreit, um hieraus den Collagenträger als weseritlichen Bestandteil des erfindungsgemäßen Mittels isolieren zu können.

Nach einer weiteren bevorzugten Ausführungsform ist der weitere Trägerbestandteil Collagen im erfindungsgemäß eingesetzten Erzeugnis ein aus natürlichen tierischen Materialien abgeleitetes Collagen. Die Herstellung dieser bevorzugt eingesetzten Collagenfasergeflechte oder Collagenschwämme ist an sich bekannt, beispielsweise aus der deutschen Offenlegungsschrift 18 11 290, der deutschen Offenlegungsschrift 26 25 289, der deutschen Patentschrift 27 34 503 und insbesondere aus der deutschen Offenlegungsschrift 32 03 957 der Anmelderin.

Nach einer weiteren bevorzugten Ausführungsform weist das erfindungsgemäße Erzeugnis nach der Komprimierung einen schwammartigen Träger mit einer Dichte von 0,005 g/cm³ bis 1,0 g/cm³, vorzugsweise 0,01 bis 0,1 g/cm³ auf. Die besagte Dichte wird nach DIN 53420 gemessen.

Nach einer weiteren bevorzugten Ausführungsform ist der Träger im erfindungsgemäßen Erzeugnis nicht verkapselt, liegt also als Pressling vor. In diesem Zusammenhang verweisen wir beispielhaft auf die Monographie Arzneimittelformenlehre von Frau Schöffling-Krause, Stuttgart, 3. Aufl., 1998, Seite 181 bis 210, und die dort beschriebenen Herstellmethoden und Maschinen, die Monographie Pharmazeutische Technologie von Bauer 1986, Seite 374 bis 413, sowie auf das Kapitel Tabletten im Buch Rudolf Voigt, Pharmazeutische Technologie für Studium und Beruf, Verlag Ullstein Mosby, Berlin 1993 S. 205 ff., und die dort beschriebenen Herstellmethoden und Maschinen hin. Die Materialzufuhr zu den Tablettenpressen wird materialgerecht modifiziert.

Nach einer weiteren bevorzugten Ausführungsform weist der Träger im erfindungsgemäßen Erzeugnis die Form einer Tablette auf. Wiederum verweisen wir beispielhaft auf die Monographie von Schöffling-Krause. Diese Tablette weist herstellungsbedingt 0,001 bis 5 g, vorzugsweise 0, 2 g bis 1 g, bezogen auf 100 g des Mittels, wenigstens eines Gleitmittels in Form eines (Matrizen)formtrennmittels auf. Beispielhaft seien hier siliconisiertes Talcum, Cetyl-Talcum, Magnesiumstearat, PEG 4000-6000, Stearinsäure, Cetylalkohol, Paraffin, Bienenwachs, hydrierte Fette und Öle und sonstige physiologisch verträgliche Formtrennmittel eingesetzt. Eine Übersicht hierüber gibt die Monographie von Rudolf Voigt im Kapitel "Tabletten". Hier ist es besonders bevorzugt als Tablette eine Oblongtablette einzusetzen.

In einer weiteren bevorzugten Ausführungsform weist die Tablette einen löslichen Überzug auf, der die Tablette überzieht. Verwiesen wird hier beispielsweise auf die Monographie von Schöffling-Krause, 1998, auf Seite 93 bis 98, sowie von Bauer Seite 397 bis 413. Dieser Überzug erfolgt üblicherweise in Mengen von 0,1 g bis 50 g, vorzugsweise 1 g bis 20 g, bezogen auf 100 g des Mittels, und kann beispielsweise aus filmbildenden, durch magensäurelösliche Lacke, beispielsweise einem Andecksirup auf Basis von Hydrogelen oder Andeckpulvern, Farbpigmentsuspensionen, einem Glättesirup oder einer Hartwachslösung oder -suspension bestehen. Weiterhin verwendet werden Filmüberzüge mit speichelresistenten, magensaftlöslichen Polymeren, beispielsweise Polyacrylaten. Weitere Filmüberzüge sind lösliche Cellulosederivate wie Hydroxypropylcellulose. Eine Übersicht über geeignete Filmbildner gibt wiederum die Monographie von Voigt im Kapitel "Dragees" ab Seite 261 ff. Weiterhin geeignet sind Überzüge nach dem Verfahren der Zuckerdragierung, wie ebenfalls aus dem Kapitel "Dragees" in der Monographie von Voigt ersichtlich.

Nach einer weiteren bevorzugten Ausführungsform ist der Träger im erfindungsgemäßen Erzeugnis verkapselt, liegt also als eine in Magensaft lösliche Kapsel vor, beispielsweise in Form einer Weichgelatinekapsel, einer Gelatinesteckkapsel oder als Kapsel mit einer modifizierten Wirkstofffreigabe. In diesem Zusammenhang verweisen wir beispielhaft auf die Monographie Arzneimittelformenlehre von Frau Schöffling-Krause, Stuttgart 1998, Seite 64 bis 81,und die dort beschrieben Herstellmethoden und Maschinen, die Monographie von Bauer, Seite 413 bis 431, sowie auf das Kapitel Kapseln im Buch von Rudolf Voigt, Pharmazeutische Technologie für Studium und Beruf, Verlag Ullstein Mosby, Berlin 1993, und die dort beschriebenen Herstellmethoden und Maschinen.

Nach einer weiteren bevorzugten Ausführungsform enthält der Träger des erfindungsgemäßen Erzeugnisses wenigstens einen Wirkstoff und/oder Zusatzstoff. Die Wirkstoffe werden zu verschiedenen Zeitpunkten bei der Herstellung den schwammartigen Trägermaterialien zugesetzt. Zusatzstoffe sind beispielsweise zugelassene Farbstoffe wie Carotinoide oder Vitamine, wie z.B. Vitamin B 2, Wirkstoffe, wie z.B. Omeprazol können ebenso zu verschiedenen Zeitpunkten, z.B. vor dem Komprimieren, den Schwämmen zugesetzt werden.

Nach einer weiteren bevorzugten Ausführungsform des erfindungsgemäßen Erzeugnisses ist der Wirkstoff in einer Matrix, Umhüllung, Einbettung und/oder einem anderen, die Freisetzung steuernden Trägermaterial enthalten. Hierdurch kommt es zu einer Wirkstofffreisetzung durch Membrandiffusion, Porendiffusion, Quellung, Erosion, Porendiffusion aus der Matrix zu einer Quellung mit Diffusion sowie zu einer Quellung mit Zerfall. Verwiesen wird hier beispielsweise auf die Monographie von R. Voigt, Kapitel "Perorale Depotarzneimittelformen" auf Bauer, Seite 533 bis 555, sowie auf Schöffling 1998, Seite 176 f und Seite 199 bis 205. Insbesondere wird im vorliegenden Falle als die Freisetzung steuerndes Trägermaterial Hydroxypropylmethylcellulose verwendet .

### Herstellung peroral verabreichbarer Erzeugnisse

Der vorliegenden Erfindung liegt weiterhin die Aufgabe zugrunde, ein Verfahren zu Herstellung des vorgenannten Erzeugnisses bereitzustellen.

Die Erfindung betrifft somit weiter ein Verfahren zur Herstellung des Erzeugnisses der vorbeschriebenen Art, wobei man einen feinporigen gefriergetrockneten Schwamm mit einer Dichte nach DIN 53420 von 0,005 bis 1 g/cm³, der gegebenenfalls vor dem Preßvorgang mit wenigstens einem Wirkstoff und/oder Zusatzstoff behandelt worden ist, gegebenenfalls in Gegenwart eines Formentrennmittels auf die Hälfte bis ein Fünfzigstel seiner Ursprungsgröße verpreßt und gegebenenfalls mit einer in Magensaft löslichen Kapsel umgibt.

Nach einer weiteren bevorzugten Ausführungsform des erfindungsgemäßen Verfahrens erfolgt die Kombination des feinporigen Schwamms mit einer Trägerschicht für mindestens einen Wirkstoff. Die Trägerschicht wird entsprechend dem Herstellungsverfahren von Schichtentabletten auf den vorkomprimierten Schwamm aufgepreßt.

Nach einer bevorzugten Ausführungsform des erfindungsgemäßen Verfahrens erfolgt die Behandlung des feinporigen Schwamms mit wenigstens einem Wirkstoff und/oder Zusatzstoff vor oder während einem wenigstens einstufigen Preßvorgang. Dies geschieht bevorzugt in der Weise, daß man die Wirk- und/oder Zusatzstoffe in an sich bekannter Weise auf den Träger in Form des Schwamms aufbringt, beispielsweise entweder pur, in einem Lösemittel gelöst oder als Dispersion in Form einer Emulsion oder Suspension.

Die Herstellung und Verpressung der Schwämme erfolgt beispielsweise nach Vorverdichtung des Schwamms nach Einlegen in eine Exzenterpresse mit einem für die Tablettenherstellung üblichen Preßwerkzeug mit Unter- und Oberstempel und geeigneter Matrize (z.B. Oblongform, 1,8 x 0,9 cm). Unter Ausstanzen erfolgt die Verpressung eines vorkomprimierten Schwammes zu einer Tablette mit einer Dicke von 4 mm. Wirkstoffe können auch vor dem Gefriertrocknungsprozeß in die Collagendispersion eingearbeitet werden.

Die vorliegende Erfindung betrifft weiterhin ein pharmazeutisches Präparat enthaltend das Erzeugnis der vorbeschriebenen oder vorhergestellten Art.

### Verwendung des peroral verabreichbaren Erzeugnisses

Als Wirkstoff im Sinne der vorliegenden Erfindung kann sowohl ein biologisch aktiver Stoff wie beispielsweise ein Pharmazeutikum eingesetzt werden, welches insbesondere während der Verweilzeit im Magen freigesetzt werden kann.

Darüber hinaus können als derartige Wirkstoffe auch Mineralien und Spurenelemente eingesetzt werden.

Dies geschieht bevorzugt in der Weise, daß man zusätzlich in diesen Träger noch nahrungsergänzende Stoffe, insbesondere Vitamine, Mineralien, Fettsäuren und/oder Ballaststoffe, zufügt oder einarbeitet. Bevorzugter Gegenstand ist daher die Verwendung des Erzeugnisses enthaltend 0,1 bis 100 Gew.-% gefriergetrocknetes Beta-1,3-Glucan aus Euglena der vorbeschriebenen oder vorhergestellten Art zur Nahrungsergänzung, insbesondere mit Vitaminen, Mineralien, Fettsäuren und/oder Ballaststoffen.

Als derartige Nahrungsergänzungsstoffe sind zunächst Vitamine zu nennen, die sich bekanntlich einerseits in fettlösliche Vitamine, wie beispielsweise Retinol, Retinsäure, Retinal, Calciferole, d.h. die D-Vitamine, die Tocopherole oder E-Vitamine und die K-Vitamine oder Phyllochinone aufteilen. Ein Mangel an A-Vitaminen bewirkt Nachtblindheit, ein Mangel an D-Vitaminen bewirkt Rachitis und ein Mangel an E-Vitaminen vermehrt die oxidative Hämolyseeignung, bewirkt hämolytische Anämien, Ödeme und eine verstärkte Erregbarkeit. Ein Mangel an K-Vitaminen bewirkt eine Störung der Blutgerinnung und der Hämorrhagien.

Eine weitere Gruppe, die erfindungsgemäß in den nahrungsergänzenden Mitteln eingesetzt werden kann, sind wasserlösliche Vitamine, wie Vitamine der B-Gruppe, wie beispielsweise Vitamin B1, das Thiamin, das Riboflavin, das Pyridoxin, die Nikotinsäure, die Corrionoide, die Folsäure und als weitere Gruppe die Ascorbinsäure bzw. das Vitamin C. Bei einem Mangel an Thiamin kommt es zur Beri-Beri-Krankheit, bei einem Mangel an Riboflavin kann sich die Cornea des Auges entzündlich verändern und es erfolgt eine erhöhte Vaskularisation. Bei einem Mangel an B6-Vitaminen kann es zu einer seborrhoischen Dermatitis, hypochromen Anämie, peripheren Neuritiden sowie zerebralen Konvulsionen kommen. Auch in der Schwangerschaft und nach einer Strahlentherapie ist ein erhöhter Bedarf an Vitamin B6 gegeben Bei einem Mangel an Nikotinsäure kommt es zur Pellagra-Krankheit, bei einer Unterversorgung an Corrinoiden kommt es zu einer perniziösen Anämie oder selbst zu einer funikulären Myelose. Bei einer Unterversorgung an Folsäure kommt es zu Problemen bei der Schwangerschaft. Bei einer Unterversorgung an Ascorbinsäure kommt es zum Skorbut und zu der Möller-Barlowschen Erkrankung.

Die Tageszufuhr an Vitaminen durch die erfindungsgemäßen Mittel ergibt sich beispielsweise aus der Empfehlung für die Nährstoffzufuhr, wie sie von der DGE zusammengestellt worden ist. Typische Tageszufuhren an Vitaminen sind beispielsweise weiterhin in der Monographie von Forth "Pharmakologie und Toxikologie" 4. Auflage, 1983, Seite 401, genannt.

Weiterer typischer Bestandteil in dem erfindungsgemäß als Nahrungsergänzungsmittel eingesetzten, oral verabreichbaren Erzeugnis sind Mineralien oder Spurenelemente, welche prophylaktisch oder therapeutisch zugeführt werden sollten. Dies sind beispielsweise Eisen, Zink, Kupfer, Mangan, Molybdän, Jod, Kobalt und Selen als essentielle Elemente für den menschlichen Körper. Bezüglich eines typischen Tagesbedarfes verweisen wir auf die vorgenannte Monographie von Forth, hier auf die Tabelle auf Seite 416.

Neben den essentiellen Elementen für den menschlichen Körper ist es in vielen Fällen auch erforderlich Calcium zuzuführen, welches nicht nur für den Knochen und Zellaufbau notwendig ist, sondern für den ganzen Körpermetabolismus. Das üblicherweise durch Nahrungsmittel vom Körper aufgenommene. Calciurn genügt nicht in allen Fällen den vorliegenden Erfordernissen. Calcium verleiht Knochen und Zähnen ihre Festigkeit.

Weiteres wesentliches Element, was erfindungsgemäß zugeführt werden kann, ist Kalium, welches eine aktive Rolle bei der Regulation des osmotischen Drucks innerhalb der Zelle spielt. Kalium ist eine Komponente des Verdauungstraktes des Magens und Darms und wird schnell resorbiert.

Weitere wesentliche, für eine Nahrungsergänzung erforderliche Komponente ist das Magnesium, welches die Muskelfunktion beeinflußt. Magnesium ist ein wesentliches Nahrungsmittel, welches in nahezu allen Zellen auftaucht und die Aktivierung von Enzymen in Bezug auf den Energiemetabolismus steuert.

Darüber hinaus können die erfindungsgemäßen Erzeugnisse auch zur Verabreichung wenigstens eines, wenigstens zum Teil löslichen, pharmakologisch wirksamen Stoffs, insbesondere mit lokaler oder systemischer Wirkung eingesetzt werden. Hierunter versteht man beispielsweise pharmakologisch wirksame Substanzen, welche auf das zentrale Nervensystem, beispielsweise als Depressiva, Hypnotika, Sedativa, Tranquilizer, Muskelrelaxantien, Antiparkinsonmittel, Analgetika, Antihypertonika, Chemotherapeutika, entzündungshemmende Mittel, Hormone, Kontrazeptiva, Sympathomimetika, Diuretika, Antiparasitenmittel, Mittel zur Behandlung der Hyperglykämie, Elektrolyte, kardiovaskuläre Mittel einwirken.

Beispiele für wasserlösliche Pharmazeutika, die durch das erfindungsgemäße Erzeugnis verzögert abgegeben werden können, schließen beispielsweise Eisensulfat, Amino-capronsäure, Kaliumchlorid, Mecamylaminhydrochlorid, Procainhydrochlorid, Amphetaminsulfat, Methamphetaminhydrochlorid, Phenmetrazinhydrochlorid, Bethanecholchlorid, Atropinsulfat, Methascopolaminbromid, Isopropamidiodid, Tridi-hexethylchlorid, Oxoprenolonhydrochlorid, Metroprolonhydrochlorid und Cimetidin-hydrochlorid ein.

Beispiele für pharmakologisch wirksame Stoffe mit begrenzter Löslichkeit in Wasser, die durch das erfindungsgemäße Erzeugnis freigesetzt werden können, sind beispielsweise Mecitinhydrochlorid, Phenoxybenzamin, Thiethylperazinmaleat, Anisindon, Reserpin, Acetolamid, Methazolamid, Chlorpropamid, Tolazimid, Chlormadinon-acetat, Aspirin, Progestin und Korticosteroide. Bezüglich der Beispiele für Arzneistoffe, die durch das erfindungsgemäße Mittel abgegeben werden können, verweisen wir auf die Pharmazeutische Stoffliste, 7. Auflage Frankfurt/M., 1989. Typische Beispiele für Arzneistoffe, die in entsprechenden Trägern verarbeitet werden, sind Acyclovir, Levodopa und Riboflavin.

Eine weitere bevorzugte Ausführungsform betrifft die Verwendung des Erzeugnisses enthaltend 0,1 bis 100 Gew.-% gefriergetrocknetes Beta-1,3-Glucan aus Euglena der vorbeschriebenen oder vorhergestellten Art zur Herstellung eines Arzneimittels oder Medizinproduktes als Magenverweilform, die über Stunden im Magen verbleibt.

Eine weitere bevorzugte Ausführungsform betriff die Verwendung des Erzeugnisses enthaltend 0,1 bis 100 Gew.-% gefriergetrocknetes Beta-1,3-Glucan aus Euglena der vorbeschriebenen oder vorhergestellten Art zur Herstellung eines Arzneimittels oder Medizinproduktes zur Verabreichung wenigstens eines, wenigstens Zum Teil löslichen, pharmakologisch wirksamen Stoffes, auch zur modifizierten Wirkstofffreisetzung

Eine besonders bevorzugte Ausführungsform betrifft die Verwendung des Erzeugnisses enthaltend 0,1 bis 100 Gew.-% gefriergetrocknetes Beta-1,3-Glucan aus Euglena der vorbeschriebenen oder vorhergestellten Art zur Herstellung eines Arzneimittels zur therapeutischen oder prophylaktischen Behandlung von Erkrankungen des Verdauungstraktes, insbesondere von Magenerkrankungen wie Magenschleimhautentzündungen.

### Parenteral verabreichbares Erzeugnis

Eine weitere bevorzugte Ausführungsform betriff die Verwendung des Erzeugnisses enthaltend 0,1 bis 100 Gew.-% gefriergetrocknetes Beta-1,3-Glucan aus Euglena der vorbeschriebenen oder vorhergestellten Art zur Herstellung eines Arzneimittels oder Medizinproduktes zur parenteralen Verabreichung, *oder* als Depot-Implantat. Hierbei handelt es sich insbesondere um Depot-Implantate oder Depot-Parentialia, mittels der biologische, insbesondere pharmazeutische Wirkstoffe über Tage, Wochen oder auch Monate verabreicht werden können, nachdem das Implanat beispielsweise subcutan oder intramusculär appliziert worden ist. Diese Implantate weisen üblicherweise Dicken im Bereich von 0,5 bis 5 mm, vorzugsweise 1 bis 4 mm auf, und können bei einem Gewicht von z.B. 50 mg bis zu 10 mg pharmazeutischen Wirkstoff pro Monat abgeben.

Die vorliegende Erfindung wird nachfolgend durch Herstellungs- und Anwendungsbeispiele erläutert und mit dem Stand der Technik verglichen. Hierin werden Teile stets als Gewichtsteile angegeben.

### Herstellungsbeispiel 1 (gefriergetrocknetes Paramylon ohne Wirksubstanz)

In einem 100 I Bioreaktor "Braun Diessel" der Firma B. Braun Melsungen wurden etwa 15 - 20 x 10⁶ *Euglena*-gracilis-Zellen (erhältlich unter der Best.-Nr 1224-5/25 über die Algensammlung in Göttingen) innerhalb von 0 bis etwa 120 Stunden (h), typischerweise innerhalb von etwa 72 h einer Fed-Batch-Kultivierung unterzogen. Dies bedeutet, daß die Zellen nicht in einem geschlossenen Batch-System mit einmaliger Nährstoffzugabe zu Beginn der Fermentation kultiviert werden, sondern in bestimmten zeitlichen Abständen wurden dem System Nährstoffe zur Supplementierung zugefügt. Dabei handelt es sich in erster Linie um Glucose, Aminosäuren und Vitamine. Auf diese Weise wird ein optimales Wachstum der Zellen, sowie eine optimale Verstoffwechselung der zugegebenen Glucose zu Paramylon gewährleistet. Die Temperatur sollte dabei im Bereich von 28 - 30°C liegen, dies ist die Temperatur, bei der *Euglena-gracilis* optimale Wachstumsraten erbringt. Weiterhin ist eine ausreichende Sauerstoffversorgung der Zellen von essentieller Bedeutung für eine optimale Paramylonsyntheserate. Diese wird während der gesamten Kultivierung kontrolliert und durch Zugabe von Sauerstoff, Entfernung des erzeugten Kohlendioxids auf Werte zwischen 0 bis 20 dm³ (= bei 760 Torr und 0 °C = NI) Luft pro min oberhalb des Kulturmediums eingestellt. Die Sauerstoffsättigung liegt typischerweise zwischen 20 und 80 %. Dies gewährleistet eine bis zu 90 %-ige Umsetzung der zugegebenen Glucosemenge zu Paramylon.

Die Ausbeuten liegen im Bereich von 12 - 18 g Paramylon / Liter Zellkultur nach einer Kultivierungszeit von etwa 72 h. Der pH-Wert während der Fermentation liegt im Bereich von 3,5 bis 6 und wird ggf. durch Säurezugabe zurücktitriert.

Im Anschluß an die Kultivierung werden die Zellen durch Zentrifugation oder einfach Sedimentation vom Kulturmedium abgetrennt und in Wasser resuspendiert. Anschließend erfolgt das Lysieren der Zellen mit Ultraschall z.B. bei 400 Watt. Das absedimentierte Paramylon wird schließlich mit einem anionischen oder nichtionischen Tensid, z.B. den Fettalkylpolyglycosiden Plantaren® oder Glucupon® (Henkel KGaA) oder dem Fettalkoholethersulfat ZETESOL® (Zschirmer & Schwarze GmbH), beides biologisch abbaubare Tenside, gewaschen und dabei mit Ultraschall behandelt. Eine andere Möglichkeit besteht im Waschen mit einem Aniontensid wie Natriumdodecylsulfat (SDS) oder im Kochen mit SDS am Rückfluß. Der Überstand wird schließlich nach dem Sedimentieren des Paramylons verworfen. Anschließend wird das Paramylon mit Wasser gewaschen und kann schließlich eingefroren oder getrocknet werden.

Das nach dem erfindungsgemäß eingesetzten Verfahren erhaltene Paramylon zeigt eine Reinheit von mehr als 99 % nach Elementaranalysen und Restproteinbestimmungen. Bei einer Kaltwäsche des Paramylons mit den genannten Tensiden liegt der Restproteingehalt bei 0,07 - 0,09 Gew-%, nach der Heißwäsche beträgt der Restproteingehalt sogar nur 0,01 - 0,03 Gew.-%.

Nach dem Waschen wird das Paramylon unter Rühren in 0,5 bis 1 M wäßriger Natriumhydroxid gelöst, so daß eine Paramylonkonzentration von etwa 5 bis 10 % in Natriumhydroxidlösung resultiert. Anschließend wird die Lösung mit Wasser auf das 5 bis 10 fache Ausgangsvolumen verdünnt und mit konzentrierter oder 1 M HCI neutralisiert. Die ausfallende gelartige Masse wird schließlich mit Wasser gewaschen und vom Kochsalz befreit.

Durch Zugabe von Wasser wird schließlich eine 0,5 bis 1 %-ige gießfähige Masse erzeugt, die auf einer glatten Oberfläche zu etwa 0,5 bis 3 cm dicken Schichten ausgegossen wird, die schließlich bei etwa - 40 °C eingefroren werden. Anschließend erfolgt die Gefriertrocknung der Schichten. Die entstehenden Folien sind im wesentlichen durch ihre Dicke sowie ihre Reißfestigkeit charakterisiert. Die Foliendicke liegt im Bereich von 50 bis 200 µm. Die Zugfestigkeit der Folien, bestimmt nach DIN EN 29 073 T3, beträgt 20 bis 25 N/mm² und die Dehnbarkeit, bestimmt nach DIN EN 29 073 T3 10 bis 20 %. Die auf diese Weise hergestellten Erzeugnisse lassen sich im wesentlichen durch ihre Dichte charakterisieren. Diese beträgt für die Matrizes etwa 0,03 bis 0,2 g/cm³. Diese Folien können parenteral eingesetzt werden.

### Herstellungsbeispiel 2 (gefriergetrocknetes Paramylon mit Collagen)

Herstellungsbeispiel 1 wurde wiederholt, allerdings vor der Gefriertrocknung eine Mischung aus 25 Gew.-% Paramylon und 75 Gew.-% Collagen unter Verwendung von Wasser als Lösemittel durch Mischen hergestellt.

### Herstellungsbeispiel 3 (gefriergetrocknetes Paramylon mit Carboxymethylcellulose und üblichen Zusätzen)

Herstellungsbeispiel 1 wurde wiederholt, allerdings vor der Gefriertrocknung eine Mischung aus 85 g Paramylon und 10 g Carboxymethylcellulose unter Verwendung von 1000 g demineralisiertem Wasser einer Gefriertrocknung unterzogen.

### Herstellungsbeispiel 4 (peroral zu verabreichendes Mittel ohne Wirksubstanz):

Ein gemäß Herstellungsbeispiel 2 hergestellter Schwamm (Länge 46 cm, Breite 8 cm, Dicke 1,3 cm) mit einem Gewicht von 12 g wird mit Hilfe einer pneumatischen Presse auf eine Breite von 1,5 cm vorkomprimiert, so daß sich ein Streifen (Länge 46 cm, Breite 1,5 cm, Dicke 1,3 cm) ergibt. Der Streifen wird abschnittsweise in eine Exzenterpresse (Tablettenpresse EK 0, Firma Korsch, Berlin) eingebracht und unter Ausstanzen des Materials mit Hilfe eines Unter- und Oberstempels und einer Matrize zu einer Oblongtablette (19 mm x 8 mm) mit jeweils vier Kerben auf Ober- und Unterseite verpreßt. Die Tabletten weisen eine Dicke von 4 mm bei einem Gewicht von etwa 400 mg auf. Die Tabletten sind nach dem Verpressen formstabil. Die Preßlinge expandieren in Wasser von 37 °C unter Aufnahme von Flüssigkeit innerhalb von maximal 5 Minuten zu einem Schwamm (1,9 cm x 0,8 cm x 8 cm). Das so erhaltene peroral verabreichbare Mittel zeigte auch nach einer Lagerung von wenigstens 2 Monaten unter Luftfeuchtigkeit keine Volumenvergrößerung.

### Herstellungsbeispiel 5 (peroral verabreichbares Mittel mit Formentrennmittel)

Wie in Herstellungsbeispiel 4 wird ein paramylonhaltiger Schwamm zu einem Streifen vorkomprimiert. Die Ober- und Unterseite des Streifens wird vor dem Komprimieren mit dem pulverförmigen Formentrennmittel Magnesiumstearat beschichtet. Im vorliegenden Beispiel wurden pro Streifen 65 mg Magnesiumstearat verwendet. Die Tabletten enthalten jeweils etwa 2 mg Formentrennmittel auf der Oberfläche. Durch das hydrophobe Formentrennmittel wird die initiale Expansion des Schwammes innerhalb der ersten Minute verzögert. In Wasser von 37 °C expandieren die Preßlinge unter Aufnahme von Flüssigkeit innerhalb von 5 Minuten zu einem Schwamm (1,9 x 0,8 x 8 cm). Das so erhaltene peroral verabreichbare Mittel zeigte auch nach 2 Monaten unter Luftfeuchtigkeit keine Volumenvergrößerung.

### Herstellungsbeispiel 6 (peroral verabreichbares Nahrungsergänzungsmittel)

Herstellungsbeispiel 4 wurde wiederholt, wobei zusätzlich 5 g natürliches Vitamin E bezogen auf 100 g des Mittels, hinzugefügt worden sind. Das so erhaltene Nahrungsergänzungsmittel zeigte auch nach einer 2-monatigen Lagerung bei Luftfeuchtigkeit keine Volumenvergrößerung.

### Herstellungsbeispiel 7 (peroral verabreichbares Mittel mit Pharmazeutikum)

Herstellungsbeispiel 4 wurde wiederholt mit der Maßgabe, daß dem feinporigen Schwammausgangsstoff zusätzlich 20 g des Arzneimittels Levodopa (L-Dioxyphenylalanin), bezogen auf 100 g des Mittels, zugesetzt worden ist. Das so erzeugte peroral pharmazeutisch wirkende Mittel zeigte auch nach einer wenigstens 2-monatigen Lagerung bei Luftfeuchtigkeit keine Volumenvergrößerung.

### Herstellungsbeispiel 8 (peroral verabreichbares Mittel mit modifizierter Wirkstofffreisetzung)

Herstellungsbeispiel 4 wurde wiederholt, wobei als modifiziertes Wirkstofffreisetzungssystem der komprimierte Schwamm mit einer weiteren Schicht aus 200 mg Hydroxypropylmethylzellulose enthaltend 100 mg Levodopa und 25 mg Benserazid (1-DL-Serin-2-(2,3,4-trihydroxbenzyl)hydrazid) als Träger für das Depotarzneimittel kombiniert wird. Die Freisetzung der Arzneistoffe erfolgt *in vitro* innerhalb von 10 Stunden. Das so erhaltene peroral verabreichbare Mittel zeigte auch nach einer Lagerung von wenigstens 2 Monaten bei Luftfeuchtigkeit keine Volumenvergrößerung.

### Anwendungsbeispiel (in vitro)

In künstlichem Magensaft nach dem US-Arzneibuch (USP XXIII) mit Pepsinzusatz wurde der in Herstellungsbeispiel 2 beschriebene Preßling bei 37 °C auf seine Abbaubarkeit innerhalb des Verdauungstraktes untersucht. Hierbei ergab sich nach 240 Minuten ein beginnender Abbau des Schwamms. Nach Wechsel des Magensaftes gegen künstlichen Darmsaft des US-Arzneibuchs (USP XXIII) mit Pankreatin zerfielen die Schwämme nach etwa 5 Stunden vollständig. Auch bei einer Einbringung in künstlichen Darmsaft erfolgte die vollständige Auflösung des Schwamms innerhalb von etwa 7 Stunden.

## Patentansprüche

1. Erzeugnis, enthaltend 0,1 bis 100 Gew.-% Beta-1,3-Glucan aus Euglena, wobei das Beta-1,3-Glucan aus Euglena erhalten worden ist durch
Kultivierung von Euglena-Zellen in einem Kulturmedium
- Abtrennung der Euglena-Zellen aus dem Kulturmedium
- Isolierung des Beta-1,3-Glucans aus Euglena aus den Euglena-Zellen
- Reinigung des Beta-1,3-Glucans aus Euglena, gegebenenfalls unter Zusatz von modifizierter Polysaccharide
sowie gegebenenfalls biologisch aktiver Wirkstoffe
- Abkühlung und anschließende Gefriertrocknung und *wobei*
- die Isolierung des Beta-1,3-Glucans aus Euglena aus den Euglena-Zellen durch Zugabe wenigstens eines Tensids erfolgt.

2. Erzeugnis nach Anspruch 1 enthaltend 3 bis 100 Gew.-% Beta-1,3-Glucan aus Euglena.

3. Erzeugnis nach Anspruch 1 oder 2, **dadurch gekennzeichnet, daß** es 0,1 bis 99 Gew.-% Beta-1,3-Glucan aus Euglena und 1 bis 99,9 Gew.-% eines weiteren Trägers, ausgewählt aus natürlichen Polysacchariden und/oder modifizierten Polysacchariden und/oder Collagen, enthält.

4. Erzeugnis nach Anspruch 1 oder 2, **dadurch gekennzeichnet, daß** es 5 bis 95 Gew.-% Beta-1,3-Glucan aus Euglena und 5 bis 95 Gew.-% eines weiteren Trägers, ausgewählt aus natürlichen Polysacchariden und/oder modifizierten Polysacchariden und/oder Collagen, enthält.

5. Erzeugnis nach Anspruch 1 bis 4, **dadurch gekennzeichnet, daß** es 1 bis 99 Gew.-% Beta-1,3-Glucan aus Euglena und 1 bis 99 Gew.-% des weiteren Trägers Collagen enthält.

6. Erzeugnis nach Anspruch 1 bis 4, **dadurch gekennzeichnet, daß** es 5 bis 95 Gew.-% Beta-1,3-Glucan aus Euglena und 5 bis 95 Gew.-% des weiteren Trägers Collagen enthält.

7. Erzeugnis nach Ansprüchen 1 bis 6, **dadurch gekennzeichnet, daß** dieses weiterhin Spinnfasern, Calziumionen, kosmetisch und pharmazeutisch aktive Wirkstoffe und micellenbildende Stoffe enthält.

8. Erzeugnis nach Anspruch 1 bis 2, **dadurch gekennzeichnet, daß** es 25 bis 75 Gew.-% Beta-1,3-Glucan aus Euglena und 25 bis 70 Gew.-% modifizierte Polysaccharide sowie 5 bis 15 Gew.-% Wasser enthält.

9. Erzeugnis nach vorstehenden Ansprüchen, **dadurch gekennzeichnet, daß** es als modifizierte Polysaccharide filmbildende Bindemittel enthält.

10. Erzeugnis nach vorstehenden Ansprüchen, **dadurch gekennzeichnet, daß** es als modifizierte Polysaccharide Celluloseether- oder -esterderivate enthält.

11. Erzeugnis nach Anspruch 10, **dadurch gekennzeichnet, daß** es zusätzlich Celluloseester, Polyester oder Polyvinylalkohol als Spinnfasern enthält.

12. Erzeugnis nach Ansprüchen 1 bis 11, **dadurch gekennzeichnet, daß** es 3 bis 30 Gew.-% Spinnfasern enthält.

13. Erzeugnis nach einem der Ansprüche 10 bis 12, **dadurch gekennzeichnet, daß** die Spinnfasern ausgewählt sind aus der Gruppe bestehend aus Celluloseestern, Polyestern, Polyamid, Polyvinylalkohol, Wolle, Baumwolle, Seide und Viskosefasern.

14. Erzeugnis nach einem der Ansprüche 8 bis 13, **dadurch gekennzeichnet, daß** die Spinnfasern eine Länge von 3 bis 30 mm und einen Titer von 1 bis 6 dtex aufweisen.

15. Erzeugnis nach den Ansprüchen 1 bis 14, **dadurch gekennzeichnet, daß** es zusätzliche pflanzliche und/oder tierische Proteine enthält.

16. Erzeugnis nach Ansprüchen 1 bis 15, **dadurch gekennzeichnet, daß** es zusätzlich Glucosaminoglucane enthält.

17. Erzeugnis nach Anspruch 16, **dadurch gekennzeichnet, daß** die Glucosaminoglucane ausgewählt sind aus der Gruppe bestehend aus Hyaluronsäure, deren Derivate und/oder Chondroitinsulfat.

18. Erzeugnis nach Ansprüchen 1 bis 17, **dadurch gekennzeichnet, daß** es micellenbildende Stoffe enthält.

19. Erzeugnis nach Anspruch 18, **dadurch gekennzeichnet, daß** die micellenbildenden Stoffe Isoparaffine sind.

20. Erzeugnis nach Ansprüchen 1 bis 6, **dadurch gekennzeichnet, daß** der Träger als biologisch aktive Wirkstoffe kosmetische und/oder pharmazeutische Wirkstoffe enthält.

21. Erzeugnis nach Anspruch 20, **dadurch gekennzeichnet, daß** es die Wirkstoffe in einer Menge von 0,1 bis 50 Gew.-%, insbesondere 3 bis 30 Gew.-% enthält.

22. Erzeugnis nach einem der Ansprüche 20 oder 21, **dadurch gekennzeichnet, daß** die Wirkstoffe in verkapselter Form vorliegen.

23. Erzeugnis nach Anspruch 22, **dadurch gekennzeichnet, daß** die Wirkstoffe in liposomalen oder liposomenähnlichen Vesikeln verkapselt sind.

24. Erzeugnis nach einem der Ansprüche 1 bis 23, **dadurch gekennzeichnet, daß** die Zusammensetzung durch Calciumionen feuchtigkeitsstabilisiert ist.

25. Erzeugnis nach Ansprüchen 1 bis 6, **dadurch gekennzeichnet, daß** es wenigstens einen komprimierten Träger enthält, wobei der Träger nach der Expansion im Magen eine schwammartige Struktur aufweist.

26. Erzeugnis nach Ansprüchen 1 bis 6 oder 25, **dadurch gekennzeichnet, daß** der Träger mit Collagenstruktur die Aminosäuren Glycin und Hydroxyprolin enthält.

27. Erzeugnis nach Ansprüchen 1 bis 6 oder 25 bis 26, **dadurch gekennzeichnet, daß** der Träger aus dem Stamm Porifera stammt.

28. Erzeugnis nach Ansprüchen 1 bis 6 oder 25 bis 31, **dadurch gekennzeichnet, daß** der vor der Komprimierung schwammartige Träger eine Dichte nach DIN 53420 von 0,005 bis 1 g/cm³ aufweist.

29. Erzeugnis nach Ansprüchen 1 bis 6 oder 25 bis 28, **dadurch gekennzeichnet, daß** der Träger nicht verkapselt ist.

30. Erzeugnis nach Ansprüchen 1 bis 6 oder 25 bis 29, **dadurch gekennzeichnet, daß** der Träger die Form einer Tablette aufweist.

31. Erzeugnis nach Anspruch 30, **dadurch gekennzeichnet, daß** die Tablette einen löslichen Überzug aufweist.

32. Erzeugnis nach Ansprüchen 1 bis 6 oder 25 bis 29, **dadurch gekennzeichnet, daß** der Träger weiterhin wenigstens einen Wirkstoff und/oder Zusatzstoff enthält.

33. Erzeugnis nach Anspruch 32, **dadurch gekennzeichnet, daß** der Wirkstoff in einer Matrix, Umhüllung, Einbettung und/oder einem anderen die Freisetzung steuernden Trägermaterial enthalten ist.

34. Erzeugnis nach Ansprüchen 1 bis 6, 25 bis 28 und/oder 31 bis 32, **dadurch gekennzeichnet, daß** der Träger verkapselt ist.

35. Verfahren zur Herstellung des gefriergetrockneten Beta-1,3-Glucans aus Euglena / Trägers nach Ansprüchen 1 bis 24 durch
- Kultivierung von Euglena-Zellen in einem Kulturmedium
- Abtrennung der Euglena-Zellen aus dem Kulturmedium
- Isolierung des Beta-1,3-Glucans aus Euglena aus den Euglena-Zellen
- Reinigung des Beta-1,3-Glucans aus Euglena, **dadurch gekennzeichnet, daß es weiterhin die**
- Umsetzung des Beta-1,3-Glucans aus Euglena, gegebenenfalls modifizierter Polysaccharide
sowie gegebenenfalls biologisch aktiver Wirkstoffe
- Abkühlung und anschließende Gefriertrocknung umfaßt und
- die Isolierung des Beta-1,3-Glucans aus Euglena aus den Euglena-Zellen durch Zugabe wenigstens eines Tensids erfolgt.

36. Verfahren nach Anspruch 35, **dadurch gekennzeichnet, daß** die Isolierung des Beta-1,3-Glucans aus Euglena aus den Euglena-Zellen ohne Zusatz organischer Lösungsmittel erfolgt.

37. Verfahren nach Ansprüchen 35 oder 36, **dadurch gekennzeichnet, daß** die Kultivierung der Euglena-Zellen als Fed-Batch-Kultivierung durchgeführt wird

38. Verfahren nach Ansprüchen 35 bis 37, **dadurch gekennzeichnet, daß** für die Isolierung des Beta-1,3-Glucans aus Euglena aus den Euglena-Zellen ein weitgehend biologisches abbaubares Tensid eingesetzt wird, welches ausgewählt ist aus nicht-ionischen oder anionischen Tensiden.

39. Verfahren zur Herstellung des Erzeugnisses nach Ansprüchen 1 bis 6 und/oder 25 bis 34, **dadurch gekennzeichnet, daß** man einen feinporigen gefriergetrockneten Schwamm mit einer Dichte nach DIN 53420 von 0,005 bis 1 g/cm³, der gegebenenfalls vor dem Preßvorgang mit wenigstens einem Wirkstoff und/oder Zusatzstoff behandelt worden ist, gegebenenfalls in Gegenwart eines Formentrennmittels auf die Hälfte bis ein Fünfzigstel, seiner Ursprungsgröße verpreßt und gegebenenfalls mit einer in Magensaft löslichen Kapsel umgibt.

40. Verfahren nach Anspruch 39, **dadurch gekennzeichnet, daß** der Preßvorgang wenigstens einstufig durchgeführt wird.

41. Verfahren nach Ansprüchen 39 oder 40, **dadurch gekennzeichnet, daß** der feinporige Schwamm mit einer Trägerschicht für mindestens einen Wirkstoff versehen wird, in dem die Trägerschicht in an sich bekannter Weise nach der Herstellung von Schichtentabletten auf den vorkomprimierten Schwamm aufgepreßt wird.

42. Pharmazeutisches Präparat, enthaltend das Erzeugnis nach Ansprüchen 1 bis 34 oder erhältlich nach Ansprüchen 35 bis 38 oder 39 bis 41.

43. Verwendung des Erzeugnisses enthaltend 0,1 bis 100 Gew.-% gefriergetrocknetes Beta-1,3-Glucan aus Euglena nach Ansprüchen 1 bis 6 zur Herstellung eines Arzneimittels oder Medizinproduktes zur parenteralen Verabreichung, *oder* als Depot-Implantat.

44. Verwendung des Erzeugnisses enthaltend 0,1 bis 100 Gew.-% gefriergetrocknetes Beta-1,3-Glucan aus Euglena nach Ansprüchen 1 bis 6 oder erhältlich nach Ansprüchen 39 bis 41 zur Nahrungsergänzung, insbesondere mit Vitaminen, Mineralien, Fettsäuren und/oder Ballaststoffen.

45. Verwendung des Erzeugnisses enthaltend 0,1 bis 100 Gew.-% gefriergetrocknetes Beta-1,3-Glucan aus Euglena nach Ansprüchen 1 bis 6 oder erhältlich nach Ansprüchen 39 bis 41 zur Herstellung eines Arzneimittels oder Medizinproduktes zur Verabreichung wenigstens eines, wenigstens zum Teil löslichen pharmakologisch wirksamen Stoffes, auch zur modifizierten Wirkstofffreisetzung.

46. Verwendung des Erzeugnisses enthaltend 0,1 bis 100 Gew.-% gefriergetrocknetes Beta-1,3-Glucan aus Euglena nach Ansprüchen 1 bis 6 oder erhältlich nach Ansprüchen 39 bis 41 zur Herstellung eines Arzneimittels zur therapeutischen oder prophylaktischen Behandlung von Erkrankungen des Verdauungstraktes.

47. Verwendung des Erzeugnisses enthaltend 0,1 bis 100 Gew.-% gefriergetrocknetes Beta-1,3-Glucan aus Euglena nach Ansprüchen 1 bis 6 oder erhältlich nach Ansprüchen 39 bis 41 zur Herstellung eines Arzneimittels oder Medizinproduktes als Magenverweilform.

## Claims

1. A product comprising 0.1% to 100% by weight of beta-1,3-glucan from *Euglena,* whereby the beta-1,3-glucan from *Euglena* is obtained by means of the
· cultivation of *Euglena* cells in a culture medium,
· separation of the *Euglena* cells from the culture medium,
· Isolation of the beta-1,3-glucan from *Euglena* from the *Euglena* cells,
· purification of the beta-1,3-glucan from *Euglena*, *of the* optionally modified polysaccharides, and if applicable, of the biologically active ingredients
· cooling and subsequent freeze-drying and wherein the isolation of the beta-1,3-glucan from *Euglena* from the Euglena-cells is performed by the addition of at least one surfactant.

2. The product according to Claim 1, comprising 3 to 100% by weight of beta-1,3-glucan from *Euglena.*

3. The product according to Claims 1 or 2, **characterized in that** it comprises 0.1% to 99% by weight of beta-2,3-glucan from *Euglena* and 1% to *99.9%* by weight of another carrier selected from among natural polysaccharides and/or modified polysaccharides and/or collagen.

4. The product according to Claims 1 or 2, **characterized in that** it comprises 5% to 95% by weight of beta-1,3-glucan from *Euglena* and 5% to 95% by weight of another carrier selected from among natural polysaccharides and/or modified polysaccharides and/or collagen.

5. The product according to Claims 1 to 4 **characterized in that** it comprises 1% to 99% by weight of beta-1,3-glucan from *Euglena* and 1% to 99% by weight of the other carrier collagen.

6. The product according to Claims 1 to 4, **characterized in that** it comprises 5% to 95% by weight of beta-1,3-glucan from *Euglena* and 5% to 9S% by weight of the other carrier collagen.

7. The product according to Claims 1 to 6, **characterized in that** it also comprises spun fibers, calcium ions, cosmetically and pharmaceutically active ingredients and micelle-forming substances.

8. The product according to Claims 1 to 2, **characterized in that** it comprises 25% to 75% by weight of beta-1,3-glucan from *Euglena* and 25% to 70% by weight of modified polysaccharides as well as 5% to 15% by weight of water.

9. The product according to the preceding Claims, **characterized in that** it comprises film-forming binders as the modified polysaccharides.

10. The product according to the preceding Claims, **characterized in that** it comprises celluloseether derivatives or cellulose-ester derivatives as the modified polysaccharides.

11. The product according to the Claim 10, **characterized in that** it additionally comprises cellulose ester, polyester or polyvinyl alcohol as spun fibers.

12. The product according to the Claims 1 to 11, **characterized in that** it comprises 3% to 30% by weight of spun fibers.

13. The product according to Claims 10 to 12, **characterized in that** the spun fibers are selected from the group consisting of cellulose esters, polyesters, polyamide, polyvinyl alcohol, wool, cotton, silk and rayon fibers.

14. The product according to one of Claims 8 to 13, **characterized in that** the spun fibers have a length of 3 to 30 mm and a titer of 1 to 6 dtex.

15. The product according to Claims 1 to 14, **characterized in that** it comprises additional plantbased and/or animal proteins.

16. The product according to Claims 1 to 15, **characterized in that** it additionally comprises glucosaminoglucanes.

17. The product according to Claim 16, **characterized in that** the glucosaminoglucanes are selected from among the group consisting of hyaluronic acid, its derivatives and/or chondroitine sulfate.

18. The product according to Claims 1 to 17, **characterized in that** it comprises micelle-forming substances.

19. The product according to Claim 18, **characterized in that** the micelle-forming substances are isoparaffins.

20. The product according to Claims 1 to 6, **characterized in that** the carrier comprises cosmetic and I or pharmaceutical active ingredients as the biologically active ingredients.

21. The product according to Claim 20, **characterized in that** it comprises the active ingredients in an amount ranging from 0.1°to 50% by weight, especially 3% to 30% by weight.

22. The product according to one of Claims 20 or 21, **characterized in that** the active ingredients are present in encapsulated form.

23. The product according to Claim 22 **characterized in that** the active ingredients are encapsulated in liposomal or liposome-like vesicles.

24. The product according to one of Claims 1 to 23, **characterized in that** the agent is stabilized against moisture by means of calcium ions.

25. The product according to Claims 1 to 6, **characterized in that** it comprises at least one compressed carrier, whereby the carrier has a sponge-like structure once it has expanded in the stomach.

26. The product according to Claims 1 to 6 or 25, **characterized in that** the carrier with the collagen structure comprises the amino acids glycine and hydroxyproline.

27. The product according to Claims 1 to 6 or 25 to 26, **characterized in that** the carrier stems from the phylum *Porifera.*

28. The product according to Claims 1 to 6 or 25 to 27, **characterized in that**, prior to its compression, the sponge-like carrier has a density in accordance with German standard DIN 53420 ranging from 0.005 to 1 g/cm³.

29. The product according to Claims 1 to 6 or 25 to 28, **characterized in that** the carrier is not encapsulated.

30. The product according to Claims 1 to 6 or 25 to 29, **characterized in that** the carrier has the form of a tablet.

31. The product according to Claim 30, **characterized in that** the tablet has a soluble coating.

32. The product according to Claims 1 to 6 or 25 to 29, **characterized in that** the carrier also comprises at least one active ingredient and / or additive.

33. The product according to Claim 32, **characterized in that** the active ingredient is contained in a matrix, shell, embedding and/or in another carrier material that controls the release.

34. The product according to Claims 1 to 6, 25 to 28, and/or 31 to 32 **characterized in that** the carrier is encapsulated.

35. A method to produce the freeze-dried paramylon carrier according to Claims 1 to 24, by means of the
· cultivation of *Euglena* cells in a culture medium,
· separation of *the Euglena* cells from the culture medium,
· Isolation of the beta-1,3-glucan from *Euglena* from the *Euglena* cells,
· purification of the beta-1,3-glucan from *Euglena* **characterized in that** it further comprises
· reaction of beta-1,3-glucan from *Euglena,* optionally modified polysaccharides and , if applicable, biologically active ingredients,
· cooling and subsequent freeze-drying and
· isolation of the beta-2,3-glucan from *Euglena* from the *Euglena-cells* using at least one surfactant.

36. The method according to Claim 35, **characterized in that** the beta-2,3-glucan from *Euglena* is isolated from the *Euglena* cells without the addition of organic solvent.

37. The method according to Claim 35 or 36, **characterized in that** it the *Euglena* cells are cultivated as fed-batch cultivation.

38. The method according to Claims 35 to 37, **characterized in that** an essentially biodegradable surfactant selected from among non-ionic or anionic surfactants is used to isolate the beta-1,3-glucan from *Euglena* from the *Euglena* cells.

39. A method for the production of the product according to Claims 1 to 6 and/or 25 to 34, **characterized in that** a fine-pore, freeze-dried sponge having a density according to German standard DIN 53420 ranging from 0.005 to 1 g/cm³, which has optionally been treated with at least one active ingredient and/or additive prior to the compression procedure, optionally in the presence of a mold-release agent, is compressed to one-half to one-fiftieth of its original size and is optionally surrounded by a capsule that is soluble in gastric juice.

40. The method according to Claim 39, **characterized in that** the compression procedure is carried out in at least one stage.

41. The method according to Claim 39 or 40, **characterized in that** the fine-pore sponge is provided with a carrier layer for at least one active ingredient, **in that** the carrier layer is compressed in a *per se* known manner onto the pre-compressed sponge alter the layer tablets have been produced.

42. Pharmaceutical composition comprising the product according to Claims 1 to 34 or obtainable according to Claims 35 to 38 or 39 to 41.

43. Use of the product comprising 0.1 to 100% by weight freeze dried beta-1,3-glucan from *Euglena* according to Claims 1 to 6 for the production of a medicament or of a medical product for parental administration, or as a depot implantat.

44. Use of the product comprising 0.1 to 100% by weight freeze dried beta-1,3-glucan from *Euglena* according to Claims 1 to 6 or obtainable according to Claims 39 to 41 as a nutritional supplement, especially of vitamins, minerals, fatty acids and/or dietary fiber.

45. Use of the product comprising 0.1 to 100% by weight freeze dried beta-1,3-glucan from *Euglena* especially according to Claims 1 to 6 or obtainable according to Claims 39 to 41 for the production of a medicament or of a medical product for purposes of administering at least one, at least partially soluble, pharmacologically active ingredient, also for modified active ingredient release.

46. Use of the product comprising 0.1 to 100% by weight freeze dried beta-1,3-glucan from *Euglena* especially according to Claims 1 to 6 or obtainable according to Claims 39 to 41 for the production of a medicament for the therapeutic or prophylactic treatment of diseases of the digestive tract.

47. Use of the product comprising 0.1 to 100% by weight freeze dried beta-1,3-glucan from *Euglena* according to Claims 1 to 6 or obtainable according to Claims 39 to 41 for the production of a medicament or of a medical product in a form that has a time of residence in the stomach.

## Revendications

1. Produit contenant entre 0,1 et 100% en poids de beta-1,3-glucane issu d'Euglena, le beta-1,3-glucane est obtenu d'Euglena par
- la culture de cellules d'Euglena dans un milieu de culture
- la séparation des cellules d'Euglena du milieu de culture
- l'isolation du beta-1,3-glucane issu d'Euglena des cellules d'Euglena
- la purification du beta-1,3-glucane issu d'Euglena, éventuellement en ajoutant du polysaccharide modifié ainsi qu'éventuellement des substances actives biologiquement
- le refroidissement et la lyophilisation ultérieure et moyennant quoi
- l'isolation du beta-1,3-glucane issu d'Euglena des cellules d'Euglena se fait par addition d'au moins un tensioactif.

2. Produit selon la revendication 1 contenant entre 3 et 100% en poids de beta-1,3-glucane issu d'Euglena.

3. Produit selon la revendication 1 ou 2, **caractérisé en ce qu'**il contient entre 0,1 et 99% en poids de beta-1,3-glucane issu d'Euglena et entre 1 et 99,9% en poids d'un autre support sélectionné parmi les polysaccharides naturels et/ou les polysaccharides modifiés et/ou le collagène.

4. Produit selon la revendication 1 ou 2, **caractérisé en ce qu'**il contient entre 5 et 95% en poids de beta-1,3-glucane issu d'Euglena et entre 5 et 95% en poids d'un autre support sélectionné parmi les polysaccharides naturels et/ou les polysaccharides modifiés et/ou le collagène.

5. Produit selon les revendications 1 à 4, **caractérisé en ce qu'**il contient entre 1 et 99% en poids de beta-1,3-glucane issu d'Euglena et entre 1 et 99% de l'autre support collagène.

6. Produit selon les revendications 1 à 4, **caractérisé en ce qu'**il contient entre 5 et 95% en poids de beta-1,3-glucane issu d'Euglena et entre 5 et 95% de l'autre support collagène.

7. Produit selon les revendications 1 à 6, **caractérisé en ce qu'**il contient, en outre, des fibres textiles, des ions de calcium, des substances actives cosmétiques et pharmaceutiques et des substances formant des micelles.

8. Produit selon les revendications 1 et 2, **caractérisé en ce qu'**il contient entre 25 et 75% en poids de beta-1,3-glucane issu d'Euglena et entre 25 et 70% en poids de polysaccharides modifiés ainsi que entre 5 et 15% en poids d'eau.

9. Produit selon les revendications précédentes, **caractérisé en ce qu'**il contient des liants filmogènes comme polysaccharides modifiés.

10. Produit selon les revendications précédentes, **caractérisé en ce qu'**il contient des dérivés d'éther ou d'ester de cellulose comme polysaccharides modifiés.

11. Produit selon la revendication 10, **caractérisé en ce qu'**il contient, en outre, de l'ester de cellulose, du polyester ou de l'alcool polyvinylique comme fibres textiles.

12. Produit selon les revendications 1 à 11, **caractérisé en ce qu'**il contient entre 3 et 30% en poids de fibres textiles.

13. Produit selon l'une des revendications 10 à 12, **caractérisé en ce que** les fibres textiles sont sélectionnées dans le groupe comprenant les esters de cellulose, les polyesters, le polyamide, l'alcool polyvinylique, la laine, le coton, la soie et les fibres de viscose.

14. Produit selon l'une des revendications 8 à 13, **caractérisé en ce que** les fibres textiles ont une longueur comprise entre 3 et 30 mm et un titre compris entre 1 et 6 dtex.

15. Produit selon les revendications 1 à 14, **caractérisé en ce qu'**il contient des protéines végétales et/ou animales supplémentaires.

16. Produit selon les revendications 1 à 15, **caractérisé en ce qu'**il contient, en outre, des aminoglucanes de glucose.

17. Produit selon la revendication 16, **caractérisé en ce que** les aminoglucanes de glucose sont sélectionnées dans le groupe comprenant l'acide hyaluronique, ses dérivés et/ou le sulfate de chondroitine.

18. Produit selon les revendications 1 à 17, **caractérisé en ce qu'**il contient des substances formant des micelles.

19. Produit selon la revendication 18, **caractérisé en ce que** les substances formant des micelles sont des isoparaffines.

20. Produit selon les revendications 1 à 6, **caractérisé en ce que** le support contient des substances actives cosmétiques et/ou pharmaceutiques comme substances actives biologiquement actives.

21. Produit selon la revendication 20, **caractérisé en ce qu'**il contient une quantité de substances actives comprise entre 0,1 et 50% en poids, de préférence entre 3 et 30% en poids.

22. Produit selon l'une des revendications 20 ou 21, **caractérisé en ce que** les substances actives se présentent sous forme encapsulée.

23. Produit selon la revendication 22, **caractérisé en ce que** les substances actives sont encapsulées dans des vésicules liposomales ou similaires à des liposomes.

24. Produit selon l'une des revendications 1 à 23, **caractérisé en ce que** l'humidité de la composition est stabilisée par des ions de calcium.

25. Produit selon les revendications 1 à 6, **caractérisé en ce qu'**il contient au moins un support comprimé, le support présentant une structure spongieuse après l'expansion dans l'estomac.

26. Produit selon les revendications 1 à 6 ou 25, **caractérisé en ce que** le support à structure de collagène contient les acides aminés glycine et hydroxyproline.

27. Produit selon les revendications 1 à 6 ou 25 à 26, **caractérisé en ce que** le support provient de la souche Porifera.

28. Produit selon les revendications 1 à 6 ou 25 à 27 **caractérisé en ce que** le support spongieux présente une densité suivant DIN 53420 comprise entre 0,005 et 1 g/cm³ avant la compression.

29. Produit selon les revendications 1 à 6 ou 25 à 28, **caractérisé en ce que** le support n'est pas encapsulé.

30. Produit selon les revendications 1 à 6 ou 25 à 29, **caractérisé en ce que** le support présente la forme d'un comprimé.

31. Produit selon la revendication 30, **caractérisé en ce que** le comprimé présente un enrobage soluble.

32. Produit selon les revendications 1 à 6 ou 25 à 29, **caractérisé en ce que** le support contient, en outre, au moins une substance active et/ou un adjuvant.

33. Produit selon la revendication 32, **caractérisé en ce que** la substance active est incluse dans une matrice, un enrobage, un emballage et/ou une autre matière support agissant sur la libération.

34. Produit selon les revendications 1 à 6, 25 à 28 et/ou 31 à 32, **caractérisé en ce que** le support est encapsulé.

35. Procédé pour la fabrication du beta-1,3-glucane issu d'Euglena/du support lyophilisé selon les revendications 1 à 24 par
- la culture de cellules d'Euglena dans un milieu de culture
- la séparation des cellules d'Euglena du milieu de culture
- l'isolation du beta-1,3-glucane issu d'Euglena des cellules d'Euglena
- la purification du beta-1,3-glucane issu d'Euglena, **caractérisé, en outre, en ce qu'**il comprend
- la conversion du beta-1,3-glucane issu d'Euglena, éventuellement du polysaccharide modifié ainsi qu'éventuellement des substances actives biologiquement,
- le refroidissement et la lyophilisation ultérieure et que l'isolation du beta-1,3-glucane issu d'Euglena des cellules d'Euglena se fait par ajout d'au moins un tensioactif.

36. Produit selon la revendication 35, **caractérisé en ce que** l'isolation du beta-1,3-glucane issu d'Euglena des cellules d'Euglena se fait sans ajout de solvants organiques.

37. Produit selon les revendications 35 ou 36, **caractérisé en ce que** la culture des cellules d'Euglena se fait sous la forme d'une culture Fed-Batch.

38. Produit selon les revendications 35 à 37, **caractérisé en ce qu'**un tensioactif largement biodégradable est utilisé pour l'isolation du beta-1,3-glucane issu d'Euglena des cellules d'Euglena, ce tensioactif étant sélectionné parmi les tensioactifs non ioniques ou anioniques.

39. Produit pour la fabrication du produit selon les revendications 1 à 6 et/ou 25 à 34, **caractérisé en ce que** l'on comprime une éponge à micropores lyophilisée dont la densité suivant DIN 53420 est comprise entre 0,005 et 1 g/cm³ et qui est éventuellement traitée avec au moins une substance active et/ou un adjuvant avant la compression, éventuellement en présence d'un lubrifiant de moule, jusqu'à une taille allant de la moitié au cinquantième de sa taille d'origine et éventuellement on l'entoure d'une capsule soluble dans le suc gastrique.

40. Produit selon la revendication 39, **caractérisé en ce que** la compression est effectuée avec au moins un étage.

41. Produit selon les revendications 39 ou 40, **caractérisé en ce que** la mousse à micropores est pourvue d'une couche support pour au moins une substance active dans laquelle la couche support est comprimée d'une manière connue en soi sur l'éponge pré-comprimée après la fabrication des comprimés en couches.

42. Préparation pharmaceutique contenant le produit selon les revendications 1 à 34 ou pouvant être obtenu selon les revendications 35 à 38 ou 39 à 41.

43. Utilisation du produit contenant entre 0,1 et 100% en poids de beta-1,3-glucane issu d'Euglena lyophilisé selon les revendications 1 à 6 pour la fabrication d'un médicament ou d'un produit médicinal pour l'administration parentérale *ou* comme implant à effet retard.

44. Utilisation du produit contenant entre 0,1 et 100% en poids de beta-1,3-glucane issu d'Euglena lyophilisé selon les revendications 1 à 6 ou pouvant être obtenu selon les revendications 39 à 41 comme complément alimentaire, en particulier avec des vitamines, des minéraux, des acides gras et/ou des substances de lest.

45. Utilisation du produit contenant entre 0,1 et 100% en poids de beta-1,3-glucane issu d'Euglena lyophilisé selon les revendications 1 à 6 ou pouvant être obtenu selon les revendications 39 à 41, pour la fabrication d'un médicament ou d'un produit médicinal pour l'administration d'au moins une substance pharmacologiquement active au moins partiellement soluble, également pour la libération modifiée d'une substance active.

46. Utilisation du produit contenant entre 0,1 et 100% en poids de beta-1,3-glucane issu d'Euglena lyophilisé selon les revendications 1 à 6 ou pouvant être obtenu selon les revendications 39 à 41, pour la fabrication d'un médicament pour le traitement thérapeutique ou prophylactique de maladies du système digestif.

47. Utilisation du produit contenant entre 0,1 et 100% en poids de beta-1,3-glucane issu d'Euglena lyophilisé selon les revendications 1 à 6 ou pouvant être obtenu selon les revendications 39 à 41, pour la fabrication d'un médicament ou d'un produit médicinal sous forme retard dans l'estomac.
